(19) Europäisches Patentamt
European Patent Office
Office européen des brevets

(11) **EP 4 686 762 A1**

(12) **EUROPEAN PATENT APPLICATION**

(43) Date of publication:
04.02.2026 Bulletin 2026/06

(21) Application number: 24191540.4

(22) Date of filing: 29.07.2024

(51) International Patent Classification (IPC):
*C12Q 1/6827* (2018.01)   *C12Q 1/6816* (2018.01)

(52) Cooperative Patent Classification (CPC):
(C-Sets available)
**C12Q 1/6827; C12Q 1/6816**   (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **ICHORtec GmbH
52353 Düren (DE)**

(72) Inventors:
• **FEDORYCH, Oleh
52062 Aachen (DE)**

• **MATYASH, Vitaly
13187 Berlin (DE)**

(74) Representative: **Fuchs Patentanwälte
Partnerschaft mbB
Tower 185
Friedrich-Ebert-Anlage 35-37
60327 Frankfurt am Main (DE)**

Remarks:
The complete document including Reference
Table(s) and the Sequence Listing(s) can be
downloaded from the EPO website

(54) **A METHOD TO CHARACTERIZE NUCLEIC ACID**

(57)   The present disclosure relates to novel optical detection methods with high sensitivity for mispairing in nucleic acids. Further, the methods allow also the detection of influences of other molecules on nucleic acid duplexes. Disclosed are also uses and applications of said methods.

Figure 7

$$\Delta E^H = E^0 - E^K$$

EP 4 686 762 A1

(52) Cooperative Patent Classification (CPC): (Cont.)

C-Sets
**C12Q 1/6816, C12Q 2522/101, C12Q 2527/125,
C12Q 2537/165, C12Q 2563/107, C12Q 2565/107;
C12Q 1/6827, C12Q 2527/125, C12Q 2537/165,
C12Q 2563/107, C12Q 2565/107**

**Description**

The present disclosure relates to novel optical detection methods with high sensitivity for mispairing in nucleic acids. Further, the methods allow also the detection of influences of other molecules on nucleic acid duplexes. Disclosed are also uses and applications of said methods.

Background of the Disclosure

Nucleic acid hybridization is a fundamental process in molecular biology where two complementary strands of nucleic acids (DNA or RNA) bind together to form a double-stranded structure, or duplex. This process is driven by the specific pairing of nucleotide bases: adenine with thymine (or uracil in RNA) and cytosine with guanine. Hybridization plays a crucial role in various biological processes, including DNA replication, transcription, and translation, and serves as the basis for numerous laboratory techniques.

Accurate nucleic acid hybridization is essential for the integrity of genetic information. Mispairings, where non-complementary bases pair, can lead to mutations that disrupt the normal function of genes. These mutations can cause a range of genetic disorders, contribute to the development of cancers, and affect the efficacy of therapeutic interventions. Therefore, identifying mispairings and other mutations is critical for understanding genetic diseases, developing diagnostic tools, and creating targeted treatments.

Several advanced technologies have been developed to detect mispairings and mutations in nucleic acids, each with unique capabilities and applications:
Next-Generation Sequencing (NGS) provides high-throughput, precise identification of mutations across entire genomes or specific target regions. Sanger Sequencing is a more traditional method for sequencing smaller regions, offering high accuracy for detecting point mutations. Allele-specific PCR is designed to amplify and detect specific mutations by using primers that match the mutated sequence. Digital PCR quantifies the exact number of mutant DNA molecules in a sample, enhancing sensitivity.

In microarrays probes are used to detect specific sequences or mutations within a sample, allowing for parallel analysis of thousands of genetic variants. Southern blotting is a more traditional method to detect specific DNA sequences within a complex mixture by hybridizing a labeled probe to the target sequence. CRISPR-Cas9 may be utilized for precise genome editing and mutation detection, enabling the identification and correction of specific genetic errors. Other techniques exploit the natural mismatch repair system to identify and quantify DNA mismatches.

Last, but not least, does Fluorescence In Situ Hybridization (FISH) make use of fluorescent probes to detect specific DNA or RNA sequences within intact cells, allowing for the visualization of chromosomal abnormalities and gene mutations.

The ability to accurately identify nucleic acid mispairings and mutations is paramount in genetics and medical research. The development of sophisticated technologies has greatly enhanced scientists capacity to detect and analyze these genetic alterations, paving the way for advancements in diagnostics, personalized medicine, and therapeutic strategies.

However, each of the prior art methods for identifying mispairings and mutations in nucleic acids do also have their own set of disadvantages:
Next-Generation Sequencing (NGS) is expensive, complex and takes long turnaround times. Snger-sequencing is limited to small regions of the genome, time-consuming, and even more costly than NGS. Allele-Specific PCR is complex in terms or primer design and can only detect known mutations. Digital PCR is again expensive and requires specialized training and handling. Microarrays are limited in their detection profile and basically restricted to pre-defined mutations. Southern Blotting is labor-intensive and has limited ability to detect low-frequency mutations. CRISPR-Cas Systems may induce unwanted off-target effects and difficult to design.

Fluorescence In Situ Hybridization (FISH) has certain resolution limits and my not detect small mutations or single nucleotide changes. It also requires skilled personnel, can be labor-intensive and expensive.

The present disclosure presents an optical method that allows for quantitative measurements of nucleic acid hybridization and mutations at room temperature and therefore overcomes several of the disadvantages mentioned before for prior art techniques. The disclosed optical method offers quantitative measurements at room temperature and could overcome many limitations, providing a faster, simpler, and more cost-effective alternative for mutation detection and analysis. This could significantly enhance the efficiency and accessibility of genetic research and clinical diagnostics.

Brief Summary of the Disclosure

In a first aspect the disclosure relates to a method for detecting nucleic acid hybridization, comprising the steps of:

a) measuring the energy ($E^0$) of a probe in a buffer, comprising the steps of

a1) providing a buffer medium;

a2) providing a nucleic acid probe with a fluorescence-label within that buffer medium;

a3) measuring the energy ($E^0$) of that system by exciting the fluorescence-tag and measuring the fluorescence of the system;

b) measuring the energy ($E^K$) of the probe hybridized to a control nucleic acid, comprising the steps of:

b1) using the same buffer medium of step a);

b2) providing the probe of step a2) and a control nucleic acid molecule which is 100% complementary to the nucleic acid probe;

b3) letting the probe and the complementary nucleic acid molecule hybridize at an temperature Ta which is from 0°C to the melting temperature of both molecules, preferably from above 0°C and up to 5°C below the melting temperature of both molecules;

b4) measuring the energy ($E^K$) of that system by exciting the fluorescence-tag and measuring the fluorescence of the system;

c) measuring the energy ($E^S$) of the probe hybridized to a sample nucleic acid, comprising the steps of:

c1) using the same buffer medium of steps a) and b);

c2) providing the probe of steps a2) and b2) and a sample nucleic acid molecule;

c3) letting the probe and the sample nucleic acid molecule hybridize at the same temperature Ta as used in step b3);

c4) measuring the energy ($E^S$) of that system by exciting the fluorescence-tag and measuring the fluorescence of the system;

and optionally,

d) repeating steps a) - c) in a buffer with a chaotropic agent; thereby receiving the energies $E'^0$, $E'^K$ and $E'^S$.

e) calculate the energy-shifts, comprising the steps of:

e1) calculate $\Delta E''$ according to the formula $\Delta E'' = E^\circ - E^K$;

e2) calculate $\Delta E^{HM}$ according to the formula $\Delta E^{HM} = E^0 - E^S$;

e3) calculate $\Delta E'^H$ according to the formula $\Delta E''' = E'^0 - E'^K$;

e4) calculate $\Delta E'^{HM}$ according to the formula $\Delta E'^{HM} = E'^0 - E'^S$;

e5) calculate $\Delta\Delta E$ according to the formula $\Delta\Delta E = |\Delta E^H| - |\Delta E^{HM}|$;

e6) optionally, calculate $\Delta\Delta E'$ according to the formula $\Delta\Delta E' = |\Delta E'^{HM}| - |\Delta E'^H|$;

wherein $|\Delta E^H| > |\Delta E^{HM}|$ - means that there is at least a single mispaired base-pair in the duplex of probe and sample nucleic acid;

wherein $\Delta\Delta E_{Dupl}$ means a decrease of the duplex melting temperature;

wherein, optionally, $|\Delta E'^H| < |\Delta E'^{HM}|$ means that in the duplex are mispaired base-pairs and

wherein $\Delta\Delta E'$ can be used to calculate the number of mispaired base-pairs by using the formula $\Delta\Delta E' \sim n\,(Tm-Ta)\,\Delta S$, where n is the number of mispaired base pairs, Tm- is the melting temperature, $T_a$ is the same temperature as of step b3), and $\Delta S$ is the entropy of a single broken hydrogen bond.

Wherein the energy shift of a mispairing event can be estimated from nearest neighbour theory (SantaLucia J (1998) A unified view of polymer, dumbbell, and oligonucleotide DNA nearest-neighbor-thermodynamics, Proceedings of the National Academy of Sciences 95(4):1460-1465).

In another aspect a method is disclosed wherein instead to the described steps c) to e), steps cc) and dd) and ee) are performed, comprising:

cc) repeating step b) in the presence of a sample molecule selected from a small molecule, peptide or protein, thereby measuring the energy $E^P$ which is the energy of the hybridized nucleic acid molecule in the presence of the sample molecule;

dd) calculate $\Delta E$ according to the formula $\Delta E = E^K - E^0$; calculate $\Delta E'$ according to the formula $\Delta E' = E'^K - E'^0$;

ee) calculate $\Delta E^P$ according to the formula $\Delta E^P = E^P - E^{0P}$; calculate $\Delta E'^P$ according to the formula $\Delta E'^P = E'^P - E'^{0P}$;

wherein the difference between the values of $\Delta E$ and $\Delta E^P$ and $\Delta E'$ and $\Delta E'^P$ indicates an interaction of the sample molecule with the duplex of the probe and the sample nucleic acid molecule.

In a third aspect the disclosure relates to the use of the disclosed methods in diagnostics outside the human or animal body, in therapy and/or in medicine.

Detailed Description of the Disclosure

As outlined before, in one embodiment the disclosure relates to a method for detecting nucleic acid hybridization, comprising the steps of:

a) measuring the energy ($E^0$) of a probe in a buffer, comprising the steps of

a1) providing a buffer medium;

a2) providing a nucleic acid probe with a fluorescence-label within that buffer medium;

a3) measuring the energy ($E^0$) of that system by exciting the fluorescence-tag and measuring the fluorescence of the system;

b) measuring the energy ($E^K$) of the probe hybridized to a control nucleic acid, comprising the steps of:

b1) using the same buffer medium of step a);

b2) providing the probe of step a2) and a control nucleic acid molecule which is 100% complementary to the nucleic acid probe;

b3) letting the probe and the complementary nucleic acid molecule hybridize at a temperature Ta which is from 0°C to the melting temperature of both molecules, preferably from above 0°C and up to 5°C below the melting temperature of both molecules;

b4) measuring the energy ($E^K$) of that system by exciting the fluorescence-tag and measuring the fluorescence of the system;

c) measuring the energy ($E^S$) of the probe hybridized to a sample nucleic acid, comprising the steps of:

c1) using the same buffer medium of steps a) and b);

c2) providing the probe of steps a2) and b2) and a sample nucleic acid molecule;

c3) letting the probe and the sample nucleic acid molecule hybridize at the same temperature Ta as used in step b3);

c4) measuring the energy ($E^S$) of that system by exciting the fluorescence-tag and measuring the fluorescence of the system;

and optionally,

d) repeating steps a) - c) in a buffer with a chaotropic agent; thereby receiving the energies $E'^0$, $E'^K$ and $E'^S$.

e) calculate the energy-shifts, comprising the steps of:

e1) calculate $\Delta E''$ according to the formula $\Delta E'' = E^0 - E^K$;

e2) calculate $\Delta E^{HM}$ according to the formula $\Delta E^{HM} = E^0 - E^S$;

e3) calculate $\Delta E'^H$ according to the formula $\Delta E''' = E'^0 - E'^K$;

e4) calculate $\Delta E'^{HM}$ according to the formula $\Delta E'^{HM} = E'^0 - E'^S$;

e5) calculate $\Delta\Delta E$ according to the formula $\Delta\Delta E = |\Delta E^H| - |\Delta E^{HM}|$ ;

e6) optionally, calculate $\Delta\Delta E$ according to the formula $\Delta\Delta E' = |\Delta E'^{HM}| - |\Delta E'^H|$;

wherein $|\Delta E^H| > |\Delta E^{HM}|$ - means that there is at least a single mispaired base-pair in the duplex of probe and sample nucleic acid;

wherein $\Delta\Delta E$ means a decrease of the duplex melting temperature;

wherein, optionally, $|\Delta E'^H| < |\Delta E'^{HM}|$ means that in the duplex are mispaired base-pairs and wherein $\Delta\Delta E'$ can be used to calculate the number of mispaired base-pairs by using the formula $\Delta\Delta E' \sim n$ (Tm-Ta) $\Delta S$, where n is the number of mispaired base pairs, $T_m$- is the melting temperature, $T_a$ is the temperature as of step b3), and $\Delta S$ is the entropy of single broken hydrogen bond.

Wherein the energy shift of a mispairing event can be estimated from nearest neighbour theory (SantaLucia J (1998) A unified view of polymer, dumbbell, and oligonucleotide DNA nearest-neighbor-thermodynamics, Proceedings of the National Academy of Sciences 95(4):1460-1465).

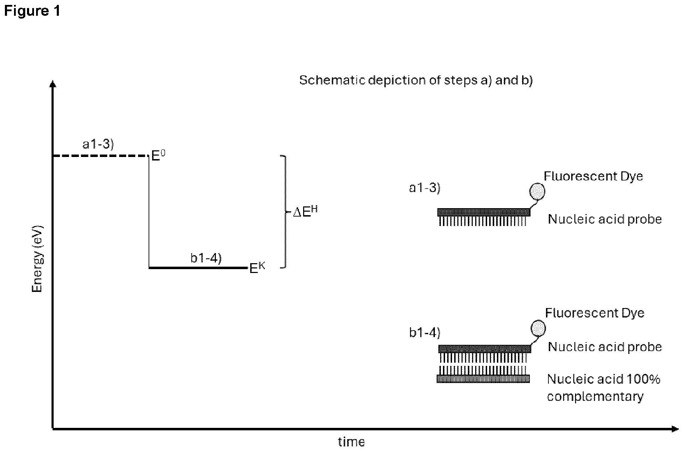

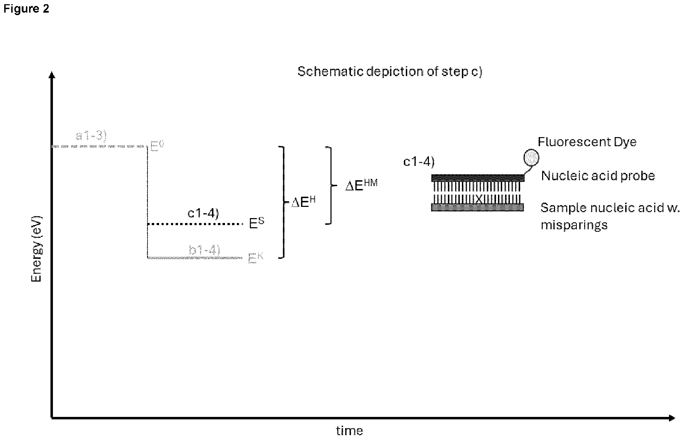

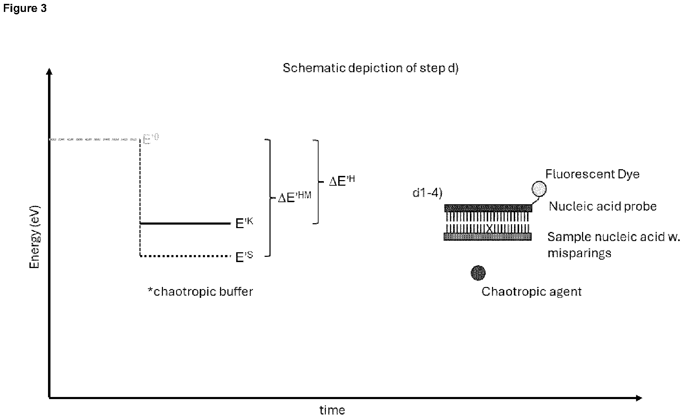

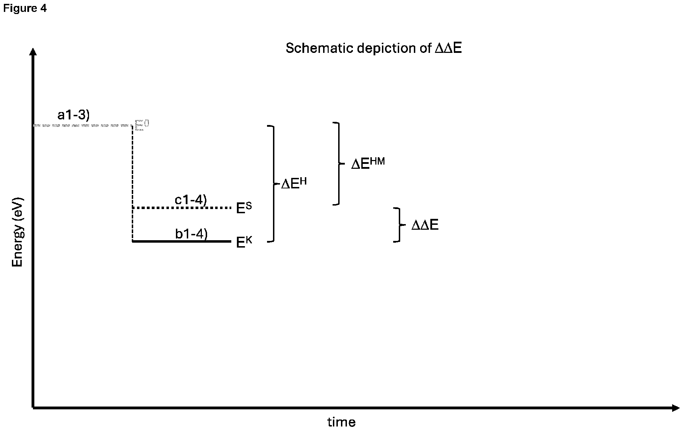

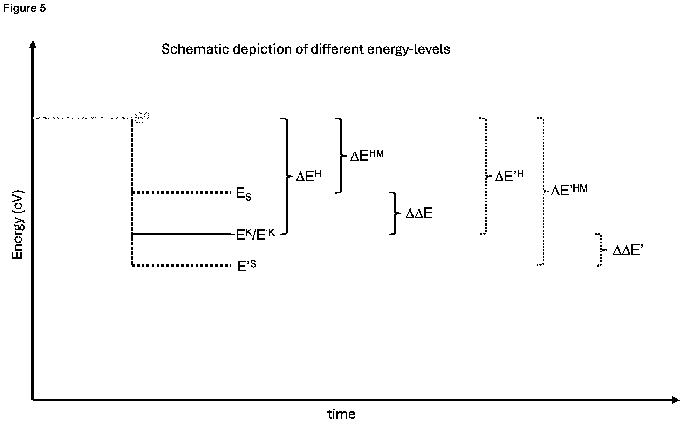

Please be also referred to figures 1 to 5 which explain the method in further detail.

The optical method as disclosed hereinunder allows for quantitative measurements of nucleic acid hybridization and mutations at room temperature could offer several significant advantages:

**Simplicity and Speed**

Rapid Results: Potential for faster analysis compared to traditional methods.

User-Friendly: Likely easier to use with minimal sample preparation.

**Cost-Effectiveness**

Lower Costs: Reduced need for expensive reagents, consumables, and equipment.

Minimal Infrastructure: Less requirement for specialized lab infrastructure.

**Qualitative and, optionally, quantitative Analysis**

Precise Measurement: Provides quantitative data on the amount of hybridized nucleic acid.

Real-Time Monitoring: Ability to monitor hybridization events in real-time.

**Room Temperature Operation**

Convenience: Simplifies experimental setup and reduces the need for temperature control.

Stability: Reduces potential for thermal degradation of samples or reagents.

**High Throughput and Scalability**

Parallel Processing: Potential for high-throughput analysis of multiple samples simultaneously.

Scalability: Easily scalable to accommodate varying sample sizes and experimental demands.

**Reduced Risk of Artifacts**

Minimized Interference: Lower risk of artifacts from complex sample preparation steps.

Direct Detection: Potential for direct detection of nucleic acid interactions without extensive amplification or labeling.

The term "probe" is used herein for a short, single-stranded piece of nucleic acid, such as DNA or RNA, that is designed to be complementary to a specific sequence of nucleic acids in a target molecule. The primary function of these probes is to detect the presence of the target sequence in a sample, such as a sample nucleic acid, by hybridizing (binding) to it through complementary base pairing. The nucleic acid probes of the present disclosure are labelled with a fluorescent molecule, they may also be referred to as fluorescent probes. The fluorescent label is a molecule that can absorb light at a particular wavelength and then emit light at a different, usually longer, wavelength. This property allows the probe to be detected and measured using fluorescence detection systems, which are highly sensitive and can provide quantitative data.

The fluorescent label is typically attached to one end of the nucleic acid probe or incorporated into its structure. When the probe binds to its target sequence, the fluorescence can be detected, indicating the presence of the target nucleic acid in the sample. Fluorescent labelling is advantageous because it allows for real-time monitoring and can provide a high degree of specificity and sensitivity.

In one embodiment the probe is a molecular beacon. That is a specific type of fluorescently labelled nucleic acid probe. It has a unique structure that allows it to fluoresce only when it is bound to its target sequence. This property makes molecular beacons particularly useful for detecting specific nucleic acid sequences in complex mixtures or for monitoring dynamic biological processes.

A molecular beacon may consist of three main parts:

- Loop Region: This is the part of the probe that contains the sequence complementary to the target nucleic acid sequence.

- Stem Region: Flanking the loop are two complementary sequences that form a short double-stranded stem. This stem keeps the probe in a closed, hairpin-like structure when it is not bound to the target.

- Fluorophore and Quencher: At opposite ends of the stem, a fluorophore and a quencher are attached. The quencher is a molecule that absorbs the fluorescence emitted by the fluorophore when they are in close proximity, thereby preventing fluorescence.

In the absence of the target sequence, the molecular beacon remains in its hairpin structure, bringing the fluorophore and quencher close together. This proximity allows the quencher to suppress the fluorescence of the fluorophore, making the beacon non-fluorescent.

When the molecular beacon encounters its target sequence, the complementary base pairing between the loop and the target sequence is stronger than the base pairing in the stem. This causes the stem to unwind and the loop to hybridize with the target. As a result, the fluorophore and quencher are separated, allowing the fluorophore to emit fluorescence. The intensity of the fluorescence is directly proportional to the amount of target sequence present, making molecular beacons useful for quantitative assays.

The typical length of the nucleic acid probe, including those used as molecular beacons, may range from 5 to 50 nucleotides, from 8 to 45 nucleotides, from 10 to 40 nucleotides, from 15 to 30 nucleotides, from 18 to 25 nucleotides or about 20 nucleotides. This length is generally sufficient to provide specific and stable hybridization with the target sequence while avoiding non-specific binding.

The design of the probe may take into account the following factors:

The sequence of the probe is carefully selected to be complementary to a unique region of the target nucleic acid, minimizing the likelihood of cross-reactivity with other sequences.

The length of the probe also influences its melting temperature (Tm), which is the temperature at which half of the probe-target duplex dissociates.

For molecular beacons specifically, the length of the loop region (the part complementary to the target) typically falls within this range, while the stem region usually consists of 4 to 6 base pairs. The exact length and composition can vary based on the specific application and the nature of the target sequence.

In one embodiment the hybridization done in steps b3), c3) and d3), as well as steps cc3) and dd3), at an temperature (Ta) which is below Tm (Ta < Tm). In some embodiments Ta is a temperature from 0°C to the melting temperature of both molecules. In some embodiments Ta is below Tm of at least 1°C, of at least 2°C, of at least 3°C, of at least 4°C, or of at least 5°C. Ta is in one embodiment at 0°C, in other embodiments above 0°C (Ta > 0°C) in order to avoid freezing of the buffer medium. It depends on the freezing point of the buffer medium how low Ta may be selected. Thus, in one embodiment Ta is > freezing point of the buffer medium. In further embodiments Ta is 1°C or more, 2°C or more, 3°C or more, 5°C or more, 10°C or more, or 15°C or more. Thus, in one embodiment Ta may be selected from freezing point of buffer to Tm, from > freezing point of buffer to < Tm, 0° to Tm, from 0°C to < Tm, from > 0°C to < Tm, from 0°C to Tm-1°C, from 0°C to Tm-2°C, from 0°C to Tm-3°C, from 0°C to Tm-4°C, from 0°C to Tm-5°C, from >0°C to Tm-1°C, from >0°C to Tm-2°C, from >0°C to Tm-3°C, from >0°C to Tm-4°C, from >0°C to Tm-5°C, from 1°C to Tm-1°C, from 2°C to Tm-2°C, from 3°C to Tm-3°C, from 5°C to Tm-4°C, or from 10°C to Tm-5°C.

The term "freezing point" is defined herein as the temperature at which a liquid turns into a solid under normal atmospheric pressure. At this temperature, the molecules of the liquid slow down enough due to a decrease in thermal energy to form a solid crystalline structure. This phase transition is a characteristic property of the substance and varies depending on the nature of the liquid.

In a further embodiment a method is disclosed wherein instead to the described steps c) to e), steps cc) and dd) and ee) are performed, comprising:

cc) repeating step b) in the presence of a sample molecule selected from a small molecule, peptide or protein, thereby measuring the energy $E^P$ which is the energy of the hybridized nucleic acid molecule in the presence of the sample molecule;

dd) calculate $\Delta E$ according to the formula $\Delta E = E^K - E^0$; calculate $\Delta E'^P$ according to the formula $\Delta E' = E'^K - E'^0$;

ee) calculate $\Delta E^P$ according to the formula $\Delta E^P = E^P - E^{0p}$; calculate $\Delta E'^P$ according to the formula $\Delta E'^P = E'^P - E'^{0P}$,

wherein the difference between the values of $\Delta E$ and $\Delta E^P$ and $\Delta E'$ and $\Delta E'^P$ indicates an interaction of the sample molecule with the duplex of the probe and the sample nucleic acid molecule.

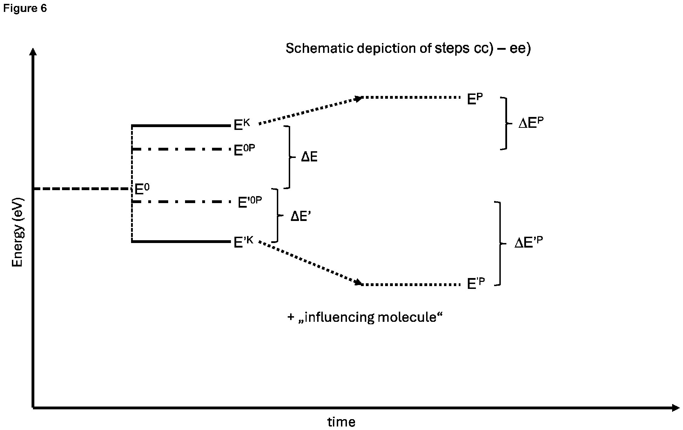

Please note also figure 6 for further information.

As outlined above the method may also be used to detect the "interference" of a molecule, selected from a small molecule (i.e. a small organic molecule), peptide and/or protein on the nucleic acid duplex. In such a case the molecule is added to the buffer and the energy shifts with respect to the solutions without the presence of the molecule are detected and calculated.

In another embodiment of the disclosure the "interference of the molecule" (such as a sample small molecule (i.e. a small organic molecule), peptide and/or protein) with the nucleic acid is selected from the group consisting of bending of the nucleic acid, looping of the nucleic acid, altering accessibility and compaction of the nucleic acid, unwinding of the nucleic acid, supercoiling the nucleic acid, and modify the nucleic acid, as well as combinations thereof.

Further, the method may also be used to define "permissive" buffer medium conditions. Permissive buffer conditions are conditions in which the energy difference between 100% complete hybridization and mispairing hybridization is either nearly zero or the mispairing event is even energetically "favoured". In those "permissive" buffer conditions therefore the mispairing event may either be statistically equal to a 100% complementary hybridization and/or may even be statistically more likely. These "permissive" buffer conditions may then be used for PCR or other nucleic acid amplification techniques in which the mispairing of the two nucleic acid sequences is preferred. Without being bound to this theory, the insight that there are buffer conditions in which nucleic acid mismatches are favoured also seems to explain why mismatched nucleic acids are more frequently amplified in some cells. This allows for the hypothesis that, at least in certain cancers and other diseases, where nucleic acid mismatches play a role, it might be explained by the presence of buffer conditions such cells in which the energetic advantage lies with incomplete hybridization rather than 100% complementary hybridization. In this sense, the disclosed method can also be adapted to measure the energetic conditions of a cell medium, and in the case of a "permissive" cell medium composition, to predict the likelihood of disease development. Thus, in one embodiment the disclosed methods may be used to predict the chances to develop a disease originating form nucleic acid mispairings, such as one selected from the group consisting of cancer, lynch syndrome (Hereditary Nonpolyposis Colorectal Cancer, HNPCC), xeroderma pigmentosum, mutations leading to genetic disorders, cystic fibrosis, sickle cell anaemia, neurodegenerative disorders such as Huntington's disease and certain forms of spinocerebellar ataxia, Fanconi anaemia, hereditary breast and ovarian cancer syndrome (such as caused by mutations in BRCA1 and BRCA2 genes), and ataxia-telangiectasia.

In one embodiment the buffer medium is water or any buffer allowing a hybridization of the nucleic acid molecules tested, i.e. which provide the necessary ionic strength and environment to facilitate the hybridization of nucleic acids to complementary strands.

In some embodiments the buffer medium may comprise:

a) a salt selected from sodium chloride (NaCl), sodium citrate, and sodium phosphate, as well as any combination thereof;

b) a detergent selected from the group consisting of sodium dodecyl sulfate (SDS), polyethylene glycol tert-octylphenyl ether (Triton X-100), nonylphenoxypoly(ethyleneoxy)ethanol (Nonidet P-40 (NP-40)), octylphenoxypolyethoxyethanol (Igepal CA-630), Polysorbate 20 (Tween 20), 3-[(3-cholamidopropyl)dimethylammonio]-1-propanesulfonate (CHAPS), N-lau-roylsarcosine sodium salt (Sarkosyl) and sodium deoxycholate (Deoxycholate), as well as any combination thereof;

c) a blocking agent selected from Denhardt's solution (comprising Ficoll, polyvinylpyrrolidone, and bovine serum albumin), salmon sperm DNA and herring sperm DNA, as well as any combination thereof;

d) a buffer such as Tris-HCl-buffer, HEPES-NaOH, saline-sodium citrate buffer, saline-sodium phosphate-EDTA buffer, as well as any combination thereof;

e) a chelating agent such as EDTA; and has a pH from 7.0 to 8.0.

In one embodiment the buffer medium is selected from the group consisting of standard SSC (Saline-Sodium Citrate) buffer, Denhardt's solution, Church buffer, formamide hybridization buffer, DIG Easy Hyb, HEPES buffer, and lysis buffer, as well as combinations thereof.

SSC (Saline-Sodium Citrate) buffer is a widely used buffer solution in molecular biology, especially in nucleic acid hybridization techniques. It provides the ionic strength and pH conditions necessary for the hybridization of DNA or RNA to complementary strands. The standard formulation for SSC buffer is as follows:

- 3 M NaCl (Sodium Chloride)

- 0.3 M Sodium Citrate (Tri-sodium citrate dihydrate)

- pH adjusted to 7.0 with HCl

Denhardt's solution is a blocking agent used in molecular biology, particularly in nucleic acid hybridization techniques, to prevent non-specific binding of probes to the membrane or other surfaces. It helps to reduce background noise and increase the specificity of the hybridization signal. The standard formulation for Denhardt's solution is as follows:

- 2% (w/v) Ficoll 400

- 2% (w/v) Polyvinylpyrrolidone (PVP)

- 2% (w/v) Bovine Serum Albumin (BSA)

These components are dissolved in distilled water to create a 100X stock solution, which can be diluted to the desired working concentration.

Church buffer, also known as Church and Gilbert buffer, is a hybridization buffer used in molecular biology to enhance the sensitivity and specificity of nucleic acid hybridization reactions. It was developed by George M. Church and Wally Gilbert. This buffer provides an optimal environment for the hybridization of DNA or RNA probes to target sequences on membranes, reducing non-specific binding and background noise. The composition of Church buffer can vary slightly depending on the specific application, but the standard formulation typically includes:

- 1% Bovine Serum Albumin (BSA)

- 1 mM EDTA (Ethylenediaminetetraacetic acid)

- 0.5 M Phosphate Buffer (pH 7.2)

- 7% SDS (Sodium Dodecyl Sulfate)

Formamide hybridization buffer is a commonly used solution in molecular biology for nucleic acid hybridization, particularly in procedures like in situ hybridization, Southern blotting, and Northern blotting. The inclusion of formamide in the buffer helps to lower the melting temperature (Tm) of nucleic acid duplexes, allowing hybridization to occur at lower temperatures, which can be crucial for preserving the integrity of nucleic acids and improving hybridization specificity.

A typical formamide hybridization buffer might include:

- 50% Formamide

- 5X SSC (Saline-Sodium Citrate) Buffer

- 0.1% SDS (Sodium Dodecyl Sulfate)

- 0.02% Polyvinylpyrrolidone (PVP)

- 0.02% Ficoll

- 0.02% Bovine Serum Albumin (BSA)

- Denhardt's Solution (sometimes included)

- 100 μg/mL denatured, sheared salmon sperm DNA (or other blocking DNA)

DIG Easy Hyb is a hybridization buffer designed for use in molecular biology applications, particularly in hybridization techniques that employ DIG (digoxigenin)-labeled probes. It is optimized to enhance the specificity and sensitivity of hybridization reactions, facilitating the detection of nucleic acids in various assays such as Southern blotting, Northern blotting, and in situ hybridization. While the exact composition of DIG Easy Hyb is proprietary, it typically includes components that promote efficient hybridization and stability of the DIG-labeled probes, such as:

- Blocking Agents: To reduce non-specific binding.

- Salts: To maintain ionic strength and stabilize nucleic acid duplexes.

- Detergents: To reduce background noise.

- Buffering Agents: To maintain optimal pH.

HEPES (4-(2-hydroxyethyl)-1-piperazineethanesulfonic acid) buffer is a zwitterionic buffering agent commonly used in biological and biochemical research due to its excellent ability to maintain a stable pH over a wide range of temperatures. In the context of nucleic acid hybridization, HEPES buffer is sometimes used to provide optimal conditions for the hybridization process, ensuring that the pH remains stable during the reaction. A typical HEPES hybridization buffer may include:

- HEPES: 10-50 mM (depending on the specific application)

- NaCl: To maintain ionic strength, typically around 0.1-1 M

- EDTA: 1-5 mM, to chelate divalent cations and prevent degradation of nucleic acids

- Detergents: Such as SDS (0.1-1%) or other non-ionic detergents, to reduce background noise

- Denhardt's Solution: Often included to block non-specific binding sites

- Formamide: 0-50%, to lower the melting temperature (Tm) of nucleic acid duplexes, facilitating hybridization at lower temperatures

- Blocking Agents: Such as sheared salmon sperm DNA or BSA, to prevent non-specific probe binding.

As lysis buffer any buffer may be used, in particular in cases in which the nucleic acid needs to be separated from other biologic material, such as in case of a cell or tissue-sample. In one embodiment the lysis buffer may comprise 50 mM Tris-HCl, 22 mM EDTA and 1.2% Triton X-100, and the pH is 7.0 - 7.5. In another embodiment the lysis buffer may comprise 150 mM NaCl, 10 mM Tris-HCl (pH 7.4) and 0.25% Triton X-100.

In one embodiment a simple Tris-HCL-Buffer may be used comprising 50 mM Tris-HCl (pH 7.4) and 100 mM HEPES-NaOH (pH 8.5).

In another embodiment of the disclosure the nucleic acid of the probe, the control nucleic acid and/or the sample nucleic acid is selected independently from each other from the group consisting of DNA (Deoxyribonucleic Acid), RNA (Ribonucleic Acid), PNA (Peptide Nucleic Acid), XNA (Xeno Nucleic Acid), LNA (Locked Nucleic Acid), morpholino oligomers, 2'O-Methyl-RNA, phosphorothioate DNA or RNA, guanosine-rich oligonucleotides, and D-amino acid-based nucleic acids, as well as combinations thereof.

In another embodiment of the disclosure the fluorescence-label is selected from the group consisting of fluorescent dyes such as DAPI, ethidium bromide, SYBR Green, Alexa Fluor dyes, and CyDyes, Quantum Dots and lanthanide-based labels, as well as combinations thereof.

In another embodiment of the disclosure the probe is excited by light in the wavelength corresponding to the absorption wavelength of the fluorescence-label.

In another embodiment of the disclosure the chaotropic agent is selected from guanidinium chloride (GuHCl), guanidinium thiocyanate (GuSCN), phenol, formamide, urea, potassium ions ($K^+$), lithium ions ($Li^+$), and magnesium ions ($Mg^{2+}$), as well as any combination thereof;

In another embodiment of the disclosure the excitation light is provided by light-source selected from the group consisting of a mercury arc lamp, a xenon arc lamp, an LED, a LASER, a metal halide lamp, a tungsten-halogen lamp, and a deuterium lamp, as well as combinations thereof.

In another embodiment of the disclosure the mispairing is a pairing selected from the group consisting of A-A, A-C, C-A, A-G, G-A, T-T, T-C, C-T, T-G, G-T, C-C, G-G, U-U, A-U, U-A, as well as combinations thereof, wherein A is adenosine, C is cytosine, T is tyrosine, G is guanine, and U is uracil.

In another embodiment of the disclosure the sample nucleic acid molecule may be derived from a cell-lysate, a biological sample such as a tissue sample, or a body fluid selected from the group consisting of blood, urine, saliva, sweat, lymph fluid, cerebrospinal fluid (CSF), gastric juice, pleural fluid, peritoneal fluid, synovial fluid (joint fluid), and sputum (phlegm), as well as combinations thereof.

In another embodiment of the disclosure the sample nucleic acid molecule is a nucleic target for use in the detection of a nucleic acid mispairing in diagnostics, research, or a therapeutic application in a biological and/or medical field.

In one aspect the present disclosure relates to a detection device as described in EP20169908.9 which is incorporated by reference hereinunder. In short, the detection device is characterized by a light source for the excitation light, and a microprocessor that is arranged for automatically receiving data from the detector. The detection device may be further characterized by comprising a beam splitter with high pass optical filter, or a dichroic mirror between the sample and the detector which differentiates the excitation energy from an emission energy to be measured by the detector. The detection device may be further characterized in that the optical element is an optical fibre. The detection device may be further characterized by comprising at least one focusing element between the light source and the sample which delivers the excitation light onto the sample. The detection device may be further characterized by comprising at least one beam collimating element between the sample and the optical element which collects the emitted light (emission energy) to the optical element.

In particular, the present disclosure concerns a method for detecting a target nucleic acid in a sample, comprising the following steps:

a. Bringing the sample in contact with a probe, that is a nucleic acid which can hybridize with the target, wherein, optionally, the probe may be labelled with a fluorescent molecule;

b. Irradiating the probe with exciting energy, preferably excitation light,

c. Detecting the emission energy from the sample, preferably emission light,

d. Calculating $\Delta E$ from the energy-difference from the sample in comparison to a standard which is the probe-target-pair of same length and chemical composition, but 100% complementary to each other allowing a perfect hybridization.

In another aspect the present disclosure pertains to a sensor vessel as described in detail in PCT/EP2022/083446 which is included by reference herein. In short, the vessel is characterized in comprising of at least a reaction chamber, a ferrule, a light conducting element, such as optical fibre (glass or plastic) with a light conducting core element and a sheath encapsulating the light conducting core element, wherein the ferrule comprises a detection buffer and a probe.

The sensor vessel combines several technical solutions:

- Safe collection and storage of biological sample (in at least one embodiment being pathogenic);

- Facilitation of a lysis of the biological sample and facilitation of the binding of the nucleic acid probe with the target nucleic acid molecule within the liquid;

- Facilitation a light conduction from the optical detection device to the complex of the a least two hybridized nucleic acid molecules (e.g. the probe and the target), thereby producing as little as possible "light pollution" in form of reflection, absorption and scattering.

In one embodiment, the sensor vessel is a "single use" or "disposable" device, which poses specific requirements regarding cost-effective production without jeopardizing the optical quality of each device.

In order to perform the disclosed method, a number of energy-levels need to be detected.

Generally, the energy difference $\Delta E$ corresponds to energy-difference of the only probe emission and the emission light of the probe-target duplex and corresponds to the hybridization energy of the at least two hybridized nucleic acid molecules; wherein a mispairing of the at least two hybridized nucleic acid molecules is characterized by a decrease or increase of energy-difference as compared to a 100% complementary control. By then adding a chaotropic substance the energy-levels may be further increased and decreased allowing for a quantitative analysis of how many base mispairings have occurred. The addition of the chaotropic substance also reduces any other artefacts which may be measured.

This finding is surprising because prior art literature suggests that mispairings should always decrease stability energy of the hybridized complex, $|\Delta E^H| > |\Delta E^{HM}|$. However, it seems that at least in certain buffer conditions, this rule does not apply, and the overall energy of the hybridization complex may be actually reduced.

The theoretical background is explained for example in the review-article (Petruska J, Sowers LC, Goodman MF. Comparison of nucleotide interactions in water, proteins, and vacuum: model for DNA polymerase fidelity. Proceedings of the National Academy of Sciences. 1986 Mar;83(6): 1559-62.)

In one embodiment the sensor vessel is configured to be used with the detection device such as described in detail in DE 10 2019 132 525.0.

In some embodiments, in particular in case of biological samples comprising intact cells, a buffer may be chosen which breaks the cells open ("lysis function") for use in the further detection reaction in order to analyze the nucleic acids within the cell, respectively, the biological sample. The biological sample may be a blood sample, a saliva sample and/or a tissue sample, or any other human body fluid or tissue sample, and therefore the cells therein need to be broken open in order to analyze the nucleic acid within. In one embodiment between at least 50% and up to 100% of the cells in a sample are "broken open", in a preferred embodiment between 60% and 99%, in a further preferred embodiment between 70 and

**EP 4 686 762 A1**

85%.

Another role of the buffer is to promote the detection reaction itself and not interfere with the detection ("detection function"), for example the buffer must promote hybridization of target molecules with the sensor probes (for example a hybridization and/or binding of the sensor probe with the target molecule) and allow the analysis to take place. For the detection function, it is important, that the buffer solution is "optically clear" at the wavelength, where the biomarker molecules are active, e.g. between 300 nm - 1100 nm, preferably between 500 nm - 700 nm.

Another role of the buffer-solution is to facilitate the binding of the biomarker with the sensor-probe.

The detection buffer of the disclosure comprises:

a) (optional) a salt selected from sodium chloride (NaCl), sodium citrate, and sodium phosphate, as well as any combination thereof;

b) a detergent selected from the group consisting of sodium dodecyl sulfate (SDS), polyethylene glycol tert-octylphenyl ether (Triton X-100), nonylphenoxypoly(ethyleneoxy)ethanol (Nonidet P-40 (NP-40)), octylphenoxypolyethoxyethanol (Igepal CA-630), Polysorbate 20 (Tween 20), 3-[(3-cholamidopropyl)dimethylammonio]-1-propanesulfonate (CHAPS), N-lauroylsarcosine sodium salt (Sarkosyl) and sodium deoxycholate (Deoxycholate), as well as any combination thereof;

c) (optional) a blocking agent selected from Denhardt's solution (comprising Ficoll, polyvinylpyrrolidone, and bovine serum albumin), salmon sperm DNA and herring sperm DNA, as well as any combination thereof;

d) a buffer such as Tris-HCl-buffer, HEPES-NaOH, saline-sodium citrate buffer, saline-sodium phosphate-EDTA buffer, as well as any combination thereof;

e) a chelating agent such as EDTA;

f) a chaotropic agent selected from guanidinium chloride (GuHCl), guanidinium thiocyanate (GuSCN), phenol, formamide, urea, potassium ions ($K^+$), lithium ions ($Li^+$), and magnesium ions ($Mg^{2+}$), as well as any combination thereof;

g) and has a pH from 7.0 to 8.0.

The concentration of the probe (e.g. the molecular probe, MB) depends on the size of the probe, the lifetime and signal intensity of the fluorochrome and the properties of the exciting light (e.g. wavelength and signal intensity of the LASER and loss of energy in the optical pathway). In some embodiments the concentration of the sensor probe was chosen to be between 0.5 μM and 20 μM, preferably between 1 μM and 10 μM, preferably between 2 μM and 5 μM, in one embodiment about 1 μM in case of a LASER with 0.25 mW of nominal power, fibre diameter of 200 μm and NA (0.22).

As fluorochrome one may use any of the following dyes: Methoxycoumarin, DyLight®, Alexa Fluor®, Brilliant Violet 421™, HiLyte Fluor™, DyLight®, Alexa Fluor®, Aminocoumarin (AMCA), BD Horizon™, Pacific Blue™, EviTagTM quantum dots-Lake Placid Blue, AMCyan, BD Horizon™, Cy2®, Chromeo™, DyLight®, Alexa Fluor®, FAM, Fluorescein Iso-thiocyanate (FITC), Evi-Tag™ quantum dots-Adirondack Green, Chromeo™, HiLyte Fluor™, Alexa Fluor® (405, 488, 514, 532, 546, 555, 568, 633, 647, 660, 680, 700, 750, 790), EviTag™, quantum dots-Catskill Green, Pacific Orange™, HEX, EviTagTM quantum dots-Hops Yellow, Cy3®, 5-TAMRA, Phycoerythrin (PE), Tetramethyl Rhodamine Isothiocyanate (TRITC), EviTag™ quantum dots-Birch Yellow, Cy3.5®, Rhodamine Red-X 570 590, PE-Dyomics®, EviTagTM quantum dots-Fort Orange, ROX, Red 613, Texas Red®, PE-Texas Red®, EviTagTM quantum dots-Maple-Red Orange, Allophycocyanin (APC), Quantum Red, Cy5®, PE-Cy5®, SureLight® P1, PE-Dyomics®, Peridinin Chlorophyll (PerCP), IRDye® 700DX, PE-Cy5.5®, APC-Cy5.5®, TruRed, APC-Cy7®, Cy7®, PE-Dyomics®, DyLight®, PE-Cy7®, IRDye® 800RS, DAPI, Hoechst 33258, Hoechst 33342, SYTOX Blue, YOYO-1, SYTOX Green, TOTO-1, TO-PRO-1, Mithramycin, SYTOX Orange, Chromomycin A3, CyTRAK Orange™, Ethidium Bromide, Propidium iodide (PI), DRAQ5™ and DRAQ7™, and/or combinations thereof.

In one embodiment system comprising the nucleic acids and the buffer medium of the present disclosure is adapted to a LASER which emits light at a wave length of at least one of the wave lengths selected from 325, 360, 405, 407, 488, 514, 532, 543, 568, 595, 633, 635 and/or 647 nm. The nominal power of the LASER may be between 1 mW and 3 W, preferably 5 mW to 1W, in some embodiments about 10 mW, 25 mW, 50 mW, 100 mW, 200 mW, 300 mW, and up to 1 W, 2 W, 3 W. Higher nominal power may lead to heating of the probe, lower nominal power usually does not generate enough signal. In general, the number of photons (and related with this the emitted optical power) which reach the carrier element should be between $2 \times 10^{15}$ and $5 \times 10^{15}$ photons/s, for example about $3.2 \times 10^{15}$ photons/s. Of course, this may be further adapted to the concentration of immobilized probes and optical activity of the used fluorochrome. In one embodiment a 532 nm LASER (diode LASER) with nominal power applied to the sample of about 0.25 mW is used.

In one embodiment the probe is a molecular beacon (i.e. nucleic acid such as for example an aptamer or RNA-probe, MB) and/or a target-binding molecule (i.e. an antigen-binding molecule, such as an antibody, fab-fragment, anticalin, or the like). In another embodiment the probe is a linear nucleic acid.

In one embodiment the method according to the disclosure preferably a peak wavelength of the detected light is defined that is specific to a hybrid of the target and the probe, and if the emitted light has the peak wavelength, it is concluded that the target is in the sample. This method is based on the finding that each target-probe hybrid has a unique wavelength of emitted light, and allows for identifying any known target, in a patient's sample.

In particular, the peak wavelength $\lambda_{em}$ may be calculated from $h * c / \lambda_{em} = h * c / \lambda_{ref} - E_0$, wherein $\lambda_{ref}$ is a reference emission of the fluorescent dye without hybridization, h is the Planck constant, c is the speed of light and $E_0$ is the hybridization energy of the target-probe hybrid, as known from Santalucia J Jr, Proc. Natl. Acad. Sci. USA 95 (1998) and calculated in terms of Nearest-Neighbor (NN) model, where change of the energy is 0.0243 eV. In some embodiments the change of the energy is between 0.01 - 0.05 eV, preferably between 0.02 - 0.03 eV, however, in some embodiment the energy may be up to 0.1 eV or even up to 0.2 eV.

In this respect it is important to note that the energy change of the peak wavelength upon hybridization and/or binding is measured (depicted as a shift between energy levels, generally called "$\Delta E$").

This measurement is not to be confused with prior art methods based for example on fluorescence excitation and/or FRET. The measurement of the present disclosure rather relies on the reduction of total free energy of the complex as compared to the single molecules, which is reduced after hybridization and/or binding.

In another embodiment the measurement allows even the differentiation between single nucleotide polymorphisms (SNPs), such as for example 1, 2, 3, 4, 5, 6, 7, 8, 9, 10, or more mispairings as long as the overall molecule still hybridizes. This allows the differentiation between different mutants or strains, e.g. different viral mutants, cancer types, and/or MRSA-strains.

In case of larger numbers of target macromolecules, such as in nano-molar-range or, preferably, micro-molar-range or, preferably, millimolar-range, as it may be the case of biomarkers found for example in blood samples, a quantitative measurement offers beneficial accuracy/precision in comparison with diagnostic methods used now (such as selective electrodes, titration methods etc.).

In one aspect the disclosure pertains to labelling a biomarker with the sensor-probe. Such a biomarker may be a nucleic acid contained in a patient's sample. According to the present disclosure in case of nucleic acids the probe is a molecular beacon. The detection buffer supports the lysis of the biological sample and the hybridization process. It is not necessary to extract the target nucleic acid (RNA or DNA) in advance since the method is very sensitive and is able to detect less than 20, preferably less than 10 copies per test. However, depending on the sample obtained, extraction of the target nucleic acid by generally known methods and/or commercially available extraction kits may be applied. In addition, when a double-stranded nucleic acid (DNA or RNA) has to be detected a strand separation can be made in advance or simultaneously with the hybridization. This strand separation may be conducted e.g. chemically could be performed simultaneously, or with UV-light or by heat treatment to be made in advance.

Detecting single nucleotide polymorphisms (SNPs) or nucleic acid mispairings under in vivo conditions opens up several significant applications in biomedical research and clinical practice.

Thus, the present disclosure encompasses the following uses and applications:

Disease Diagnosis and Personalized Medicine

Genetic Disorders: Identifying SNPs associated with genetic diseases allows for early diagnosis and personalized treatment plans.

Cancer: Detecting SNPs linked to cancer susceptibility or drug resistance helps in designing targeted therapies.

Infectious Diseases: Identifying genetic variations in pathogens helps in tracking outbreaks and selecting appropriate treatments.

Pharmacogenomics

Drug Response: SNPs influencing drug metabolism or efficacy can guide personalized drug selection and dosage adjustments.

Adverse Drug Reactions: Predicting susceptibility to adverse drug reactions based on genetic variations improves patient safety.

Population Genetics and Evolutionary Studies

Studying SNP frequencies in populations provides insights into genetic diversity, migration patterns, and evolutionary relationships.

Identifying genetic adaptations to environmental pressures helps in understanding human evolution and adaptation.

Forensic Analysis

Using SNPs for individual identification and forensic DNA profiling enhances accuracy and reliability in criminal investigations and paternity testing.

Monitoring Treatment Efficacy

Monitoring changes in SNP profiles during treatment helps in assessing treatment response and predicting outcomes.

Non-Invasive Prenatal Testing (NIPT)

Detecting fetal SNPs from maternal blood allows for non-invasive prenatal screening for genetic disorders.

Environmental Monitoring and Agriculture

Assessing genetic diversity and health of wildlife populations or agricultural crops using SNP analysis contributes to conservation and breeding programs.

In summary, the ability to detect SNPs and nucleic acid mispairings in vivo has transformative implications across medicine, research, and beyond, enabling more precise diagnostics, treatments, and understanding of genetic diversity and disease mechanisms.

In another aspect of the disclosure the method can be applied to analyse the influence of other macromolecules such as peptides, proteins, carbohydrates or lipids on nucleic acid duplexes in a patient. The inventive method may be used both for predictive a well as diagnostic purposes.

In case of larger numbers of target macromolecules, such as in nano-molar-range or, preferably, micro-molar-range or, preferably, millimolar-range, as it may be the case of biomarkers found for example in blood samples, a quantitative measurement becomes especially feasible.

As used herein the term "optically clear" refers to a material which shows a transmissibility of the light at least in the detection wavelength of 500 to 700 nm of at least 95% or more, preferably of 98% or more, most preferably of 99% or more, such as more than 99.9%, more than 99.95%, more than 99.99%. Optically clear also implies that the material does not show any fluorescent effects neither in itself, nor on the probe alone or bound to the target molecule (i.e. the biomarker). Furthermore, "optically clear" materials preferably do not absorb and/or scatter the light.

As used herein the term "optically inactive" refers to a material which shows weak (a few %, i.e. 0.1%, 0.5%, 1%, less than 5%, less than 2.5%) to no transmissibility of the light at least in the in the detection wavelength of 500 to 700 nm. For example, a transmission of 0.01% or less, preferably of 0.001% or less, most preferably of 0.0001% and even better less than the detection limit. Furthermore, the term "optically inactive" as used herein refers to a material which shows no other optical effects such as luminescence or phosphorescence upon excitation with light. According to the present disclosure "optically inactive" material is used to avoid any "light contamination", e.g. any light which comes from sources apart from the sensor-probe.

**Definitions**

As used herein, "nucleic acid" or "polynucleic acid" refers to the order or sequence of nucleotides along a strand of nucleic acids. The nucleic acid sequence may be single-stranded or double-stranded or contain portions of both double-stranded and single-stranded sequences. The nucleic acid sequence may be composed of DNA, both genomic and cDNA, RNA or DNA/RNA hybrid.

As used herein, "macromolecule" refers to any large biomolecule including nucleic acids, peptides, proteins, carbohydrates or lipids.

As used herein, "peptide" refers to short chains of between two and fifty amino acids, linked by peptide bonds. Chains of fewer than ten or fifteen amino acids may also be called oligopeptides, and include dipeptides, tripeptides, and tetrapeptides.

As used herein, "protein" refers to large biomolecules that are comprised of one or more long chains of amino acid residues. Proteins perform a vast array of functions within organisms, including catalysing metabolic reactions, DNA replication, responding to stimuli, providing structure to cells and organisms, and transporting molecules from one location to another. Proteins differ from one another primarily in their sequence of amino acids, which is dictated by the nucleotide sequence of their genes, and which usually results in protein folding into a specific 3D structure that determines its activity.

As used herein, "carbohydrate" refers to a biomolecule consisting of carbon (C), hydrogen (H) and oxygen (O) atoms, usually with a hydrogen-oxygen atom ratio of 2:1 (as in water) and thus with the empirical formula $C_m(H_2O)_n$ (where $m$ may or may not be different from $n$). However, not all carbohydrates conform to this precise stoichiometric definition (e.g., uronic acids, deoxy-sugars such as fucose), nor are all chemicals that do conform to this definition automatically classified as carbohydrates (e.g. formaldehyde). It is used herein primarily as a synonym of saccharide, a group that includes sugars, starch, and cellulose.

As used herein, "lipid" refers to a macro biomolecule that is soluble in nonpolar solvents. Nonpolar solvents are typically hydrocarbons used to dissolve other naturally occurring hydrocarbon lipid molecules that do not (or do not easily) dissolve in water, including fatty acids, waxes, sterols, fat-soluble vitamins (such as vitamins A, D, E, and K), monoglycerides, diglycerides, triglycerides, and phospholipids. As such the term encompasses fatty acids, glycerolipids, glycerophospholipids, sphingolipids, sterols, prenols and saccharolipids.

As used herein the term "sensor-probe" is used herein for any molecule which is able to specifically bind to a target molecule, i.e. via hybridization and/or other binding modes, such as for example via van-der-Waals-bonds, covalent bonds and/or hydrogen bonds. Thus, the term "sensor-probe" without further definition encompasses "molecular beacons", or "(molecular beacon) probes", as well as "target-binding molecules" (such as for example antibodies).

As used herein, the terms "target/probe molecule complex" or "complex" are used in a general manner for any group of two or more associated molecules. For example, such an association may be the hybridization of the molecular beacon and the target nucleic acid and/or the binding of the target-binding molecule and the target macromolecule. The complex is excited by light and the emission is detected in the analysing device.

As used herein, "molecular beacons" are oligonucleotides that can report the presence of specific nucleic acids in homogenous solutions. Molecular beacons are hairpin-shaped molecules with a fluorochrome. This is a novel nonradioactive method for detecting specific sequences of nucleic acids. They are useful in situations where it is either not possible or desirable to isolate the molecular beacon-target hybrids from an excess of the molecular beacons.

A typical molecular beacon is 25-40 nucleotides long. The middle nucleotides are complementary to the target DNA or RNA and do not base pair with one another, while the five to seven nucleotides at each terminus are complementary to each other rather than to the target DNA.

A typical molecular beacon structure can be divided in 4 parts: 1) loop, an 18-30 base pair region of the molecular beacon that is complementary to the target sequence; 2) stem formed by the attachment to both termini of the loop of two short (5 to 7 nucleotide residues) oligonucleotides that are complementary to each other; 3) 5' fluorescent dye at the 5' end of the molecular beacon, a fluorescent dye is covalently attached; 4) 3' quencher (non-fluorescent) dye that is covalently attached to the 3' end of the molecular beacon. When the beacon is in closed loop shape, the quencher resides in proximity to the fluorescent dye, which results in quenching the fluorescent emission of the latter. If the nucleic acid to be detected is complementary to the strand in the loop, the event of hybridization occurs. The duplex formed between the nucleic acid and the loop is more stable than that of the stem because the former duplex involves more base pairs. This causes the separation of the stem and hence of the fluorescent dye and the quencher. Once the fluorescent dye is no longer next to the quencher, illumination of the hybrid with light results in the fluorescent emission. The presence of the emission reports that the event of hybridization has occurred and hence the target nucleic acid sequence is present in the test sample. The fluorescent dye of the molecular beacon may be any suitable fluorescent dye, for example ABI dyes (e.g. FAM™, HEX™, TET™, JOE™, ROX™, CAL Fluor™ Red 610), cyanine dyes (e.g. Yakima Yellow or ATTO) or molecular dyes (e.g. ALEXA-fluor or BODIPY dyes). The quencher of the molecular beacon may be any suitable quencher, for example TAM, BHQ1, BHQ2, DAB, Eclip, BBQ650. Molecular beacons are synthetic oligonucleotides whose preparation is well documented in the literature (Tyagi S; Kramer FR, (1996). "Molecular beacons: probes that fluoresce upon hybridization". Nat. Biotechnol. 14 (3): 303-308). Further, the molecular beacon design is within the skill of a molecular biologist and there are many bioinformatics tools for this purpose available (e.g. Beacon DesignerTM). In addition, molecular beacons are commercially available for many target sequences (e.g. Eurofins Genomics, Ebersberg, Germany; Integrated DNA Technologies, Inc., Coralville, Iowa, USA).

In the context of this disclosure, "hybridization" means hydrogen bonding, which may be Watson-Crick, Hoogsteen or reversed Hoogsteen hydrogen bonding, between complementary nucleoside and/or nucleotide bases. Hybridization takes place under stringent or non-stringent conditions. As used herein, "stringent" refers to the conditions, i.e. temperature, buffer composition or ionic strength under which hybridization between polynucleotides occurs. These conditions depend mainly on the composition and complexity of the target nucleic acid and length of the molecular beacon probe.

For the hybridization temperature conditions the "$T_m$" (melting temperature) of the nucleic acids has to be considered. "$T_m$" means under specified conditions the temperature at which half of the nucleic acid sequences are disassociated and half are associated. Generally, suitable hybridization conditions may be easily determined by a person skilled in the art.

"Complementary" as used herein, refers to the capacity for pairing between two nucleotides. For example, if a nucleotide at a certain position of an oligonucleotide is capable of hydrogen bonding with a nucleotide at the same position of a DNA or RNA molecule, then the oligonucleotide and the target DNA or RNA are considered to be complementary to each other at that position. For example, the sequence 5'-A-C-T-3' is complementary to the sequence 3'-T-G-A-5'.

Complementarity may be partial, in which only some of the nucleotides match according to base pairing, or complete, where all the nucleotides match according to base pairing. For purposes of the present disclosure "substantially complementary" refers to 90% or greater identity over the length of the target base pair region. The complementarity can also be 45, 50, 60, 70, 75, 80, 85, 90, 91, 92, 93, 94, 95, 96, 97, 98, 99, or 100% complementary, or any amount below or in between these amounts. In other words, the oligonucleotide and the target DNA or RNA are complementary to each other when a sufficient number of corresponding positions in each molecule are occupied by nucleotides which can build a hydrogen bond with each other. Thus, "specifically hybridizable" and "complementary" are terms which are used to indicate a sufficient degree of complementarity or precise pairing such that stable and specific binding occurs between the molecular beacon probe and the DNA or RNA target. It is understood in the art that the molecular beacon probe does not need to be 100% complementary to that of its target nucleic acid to be specifically hybridizable. "Oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly.

As used herein, "target-binding molecule" refers to a molecule, preferably a protein, which is able to specifically bind to a target macromolecule (such as a peptide, protein, carbohydrate or lipid) in a sample. Such a target-binding molecule may be selected from the group comprising antibody, antibody-fragment, Fab-fragment, anticalin protein, DARPin, nanoCLAMP, affilin, affimer, affitin, alphabody, avimer, Kunitz domain peptide, monobody, intrabody, microantibody, single-chain variable fragment (scFv), and combinations thereof. Target-binding molecules typically bind to unique surface patterns on the target macromolecule, sometimes referred to as "epitope".

As used herein, "specifically binding" refers to the binding of the target-binding molecule to the target macromolecule (such as a peptide, protein, carbohydrate or lipid) in a sample with a high specificity. The fewer ligands a protein can bind besides the desired target, the greater is its specificity. Specificity describes the strength of binding between a given protein and ligand. This relationship can be described by a dissociation constant ($K_D$), which characterizes the balance between bound and unbound states for the protein-ligand system. $K_D$ is the equilibrium dissociation constant, a calculated ratio of $K_{off}/K_{on}$, between the target-binding molecule and its target. The association constant ($K_{on}$) is used to characterise how quickly the target-binding molecule binds to its target. The dissociation constant ($K_{off}$) is used to measure how quickly a target-binding molecule dissociates from its target. The specificity of the target-binding molecule is at least micromolar ($\mu$M, $K_D$ between $10^{-4}$ to $10^{-6}$), more preferably nanomolar (nM, $K_D$ between $10^{-7}$ to $10^{-9}$), even more preferably picomolar (pM, $K_D$ between $10^{-10}$ to $10^{-12}$), and most preferably femtomolar (fM, $K_D$ between $10^{-13}$ to $10^{-15}$).

"Subject" or "patient" as used herein refers to a living organism, such as a mammalian individual, including murines, cattle, simians and humans. Preferably, the patient is a human.

"Biological sample" or just "sample" as used herein refers to a biological sample encompassing liquid and solid samples. Liquid samples encompass saliva, sputum, sweat, urine, nasal secretion, bronchoalveolar lavage fluid, laryngo-pharyngeal scrape test, vaginal secretion, blood liquids (e.g. serum, plasma) and cerebrospinal fluid (CSF). Solid samples encompass tissue samples such as tissue cultures or biopsy specimen. A preferred patient's sample is sputum. The patient's sample will be collected with the disposable pipette as described below.

In a particular preferred embodiment of the present disclosure, the method is used for detecting a biomarker (including pathogens) in a patient's sample.

"Biomarker" as used herein, is a measurable indicator of some biological state or condition. Biomarkers are often measured and evaluated using blood, urine, or soft tissues to examine normal biological processes, pathogenic processes, or pharmacologic responses to a therapeutic intervention. As such the disclosure pertains to labeling a biomarker such as a macromolecule (such as nucleic acids, peptides, proteins, carbohydrates or lipids) contained in a patient's sample (e.g. blood- sample, liquor-sample and/or saliva sample comprising the target macromolecule) with a sensor-probe. For simplification the definition of "biomarker" within this application encompasses also pathogens.

"Pathogens" as used herein, is any organism that can produce disease. A pathogen may also be referred to as an infectious agent, or simply a germ. Typically, the term is used to describe an infectious microorganism or agent, such as a virus, bacterium, protozoan, prion, viroid, or fungus.

As used herein, "nucleic acid" or "polynuclec acid" refers to the order or sequence of nucleotides along a strand of nucleic acids. The nucleic acid sequence may be single-stranded or double-stranded or contain portions of both double-stranded and single-stranded sequences. The nucleic acid sequence may be composed of DNA, both genomic and cDNA, RNA or DNA/RNA hybrid.

The fluorescent dye attached (optionally covalently bound) to the probe may be any suitable fluorescent dye, for example ABI dyes (e.g. FAMTM, HEXTM, TETTM, JOETM, ROXTM, CAL FluorTM Red 610), cyanine dyes (e.g. Yakima Yellow or ATTO) or molecular dyes (e.g. ALEXA-fluor or BODIPY dyes). The quencher of the probe may be any suitable quencher, for example TAM, BHQ1, BHQ2, DAB, Eclip, BBQ650.

In the context of this disclosure, "hybridization" means hydrogen bonding, which may be Watson-Crick, Hoogsteen or reversed Hoogsteen hydrogen bonding, between complementary nucleoside or nucleotide bases. Hybridization takes place under stringent or non-stringent conditions.

As used herein, "stringent" refers to the conditions, i.e. temperature, buffer composition or ionic strength under which hybridization between polynucleotides occurs. These conditions depend mainly on the composition and complexity of the target nucleic acid and length of the probe.

For the hybridization temperature conditions the "Tm" (melting temperature) of the nucleic acids has to be considered. "Tm" means under specified conditions the temperature at which half of the nucleic acid sequences are disassociated and half are associated. Generally, suitable hybridization conditions may be easily determined by a person skilled in the art.

"Complementary" as used herein, refers to the capacity for pairing between two nucleotides. For example, if a nucleotide at a certain position of an oligonucleotide is capable of hydrogen bonding with a nucleotide at the same position of a DNA or RNA molecule, then the oligonucleotide and the target DNA or RNA are considered to be complementary to each other at that position.

For example, the sequence 5'-A-C-T-3' is complementary to the sequence 3'-T-G-A-5'. Complementarity may be partial, in which only some of the nucleotides match according to base pairing, or complete, where all the nucleotides match according to base pairing. For purposes of the present disclosure "mispairing" refers to a situation where at least two bases are not complementary to each other, e.g. where the base-pair is C-A, A-C, T-G, G-T, U-A or A-U.

The term "substantially complementary" refers to 90% or greater identity over the length of the target base pair region. The complementarity can also be 45, 50, 60, 70, 75, 80, 85, 90, 91, 92, 93, 94, 95, 96, 97, 98, 99, or 100% complementary, or any amount below or in between these amounts. In other words, the oligonucleotide and the target DNA or RNA are complementary to each other when a sufficient number of corresponding positions in each molecule are occupied by nucleotides which can build a hydrogen bond with each other. Thus, "specifically hybridizable" and "complementary" are terms which are used to indicate a sufficient degree of complementarity or precise pairing such that stable and specific binding occurs between the probe and the DNA or RNA target. It is understood in the art that the probe does not need to be 100% complementary to that of its target nucleic acid to be specifically hybridizable.

"Oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly.

"Subject" or "patient" as used herein refers to a mammalian individual, including murines, cattle, simians and humans. Preferably, the patient is a human.

"Sample" as used herein refers to a biological sample encompassing liquid and solid samples. Liquid samples encompass saliva, sputum, sweat, urine, nasal secretion, bronchoalveolar lavage fluid, laryngo-pharyngeal scrape test, vaginal secretion, blood liquids (e.g. serum, plasma) and cerebrospinal fluid (CSF). Solid samples encompass tissue samples such as tissue cultures or biopsy specimen. A preferred patient's sample is sputum. The patient's sample will be collected with a disposable device as described hereinunder.

In a particular preferred embodiment of the present disclosure, the method is used for detecting a virus in a patient's sample. "Virus" or "viral" means any single- or double-stranded DNA or RNA virus. In particular, Human Immunodeficiency Virus (HIV), Zika, MERS-Corona, SARS-CoV-1, Sars-CoV-2, Hepatitis B Virus (HBV), Hepatitis C Virus (HCV), Human Lyphotropic Virus-type 1-4 (HTLV-1 - 4), Epstein-Barr Virus (EBV), Human Papilloma Virus (HPV), Influenza A, B or C, Non-Influenza Respiratory Viruses (NIRVs, e.g. respiratory syncytial virus, parainfluenza virus, rhinovirus, metapneumovirus), norovirus, Ebola virus, Reovirus, Herpes virus (simplex 1, 2 + B, 5, 6, 7, 8), Pox virus, FSME virus, Dengue virus, Rubivirus, Varizella zoster virus and Cytomegalievirus.

In a method according to the disclosure, the target nucleic acid may in one embodiment be a virus RNA. This method provides for screening large numbers of patients for infection, in an extremely reduced time scale, compared to the state of the art. The virus may in particular be a coronavirus, further in particular SARS-CoV-2. The method is thus applicable in a recent worldwide pandemic situation. In a method according to the disclosure, the excitation light may in particular be a laser beam. This method allows for limiting the excitation light to the excitation wavelength of the fluorescent dye, and thus for avoiding side effects induced by other wavelengths.

Types of nucleic acids disclosed hereinunder:

Genomic DNA: Detecting specific genes, mutations, or overall genome integrity.

Mitochondrial DNA (mtDNA): Used for studying maternal lineage, certain diseases, and forensic analysis.

Plasmid DNA: Common in bacterial studies and biotechnology applications.

Viral DNA: Detection of DNA viruses like herpesvirus, human papillomavirus (HPV), and adenovirus.

mRNA (messenger RNA): Reflects gene expression levels; used in gene expression studies and diagnostics.

rRNA (ribosomal RNA): Important for detecting and quantifying ribosomes; used in microbial identification and taxonomy.

tRNA (transfer RNA): Less common in diagnostics but crucial for understanding translation processes.

miRNA (microRNA): Small non-coding RNA molecules involved in gene regulation; used in cancer and other disease diagnostics.

siRNA (small interfering RNA): Used in gene silencing studies and therapeutic applications.

Viral RNA: Detection of RNA viruses like influenza, HIV, SARS-CoV-2 (the virus responsible for COVID-19), hepatitis C virus (HCV), and Zika virus.

lncRNA (long non-coding RNA): Involved in gene regulation; studied in various diseases including cancer.

snRNA (small nuclear RNA): Involved in RNA splicing; studied in certain genetic disorders.

Brief Description of the Drawings

**Figure 1** - shows the energy measurements according to steps a) and b) which is the energy-shift upon hybridization of the probe with a 100% complementary control.

**Figure 2** - shows the energy measurements according to step c) which is the energy-shift upon hybridization of the probe with a sample nucleic acid, which for example has one mismatch.

**Figure 3** - shows the energy measurements according to step d) which is the repetition of the measurement b) in the same buffer but comprising a chaotropic agent.

**Figure 4** - shows the different energy-differences which can used in order to qualitatively define that in the sample nucleic acid exists a mismatch as compared to the control.

**Figure 5** - shows the different energy-differences which can used in order to quantitatively define how many mismatches are present in the sample nucleic acid as compared to the control.

**Figure 6** - shows the alternative method which may be used in order to measure the influence of a molecule, such as small molecule, peptide or protein, on the duplex formed of the at least two nucleic acids present in the buffer medium.

**Figure 7** - Shows the energy shift upon the formation of a duplex structure.

**Figure 8** - Shows a sample of a detection device which may be used in order to measure the energy-shifts in the sample. Wherein 1. light source (laser/LED/...), 2. excitation irradiation, 3. beam splitter, 4. Mirror, 5. micro-objective, 6. sample plate, 7. Sample, 8. xyz translation stage, 9. emission (fluorescence), 10. emission filter (fluorescence filter), 11. coupling lens, 12. spectrometer (detector).

**Figure 9** - shows a real depiction of the energy-shifts in buffer (A) and in a chaotropic substance guanidinium thiocyanate (B). Molecular probe (SEQ ID No. 1) 5'-AGA CCA GAA GAT CAG GAA CTC TA -3' (Rhodamine 6G at 5'), Target without polymorph nucleotides: (SEQ ID No. 2) 5'-TA GAG TTC CTG ATC TTC TGG TCT-3', Target with polymorph nucleotide: (SEQ ID No. 3) 5'-TA GAG TTC CTG ATT TTC TGG TCT-3'.

**Figure 10** - shows the energy levels in buffers with different concentrations of a chaotropic agent (urea). a) urea 0 mM, b) urea 1 mM, c) urea 2 mM, d) urea 3 mM, and e) d) urea 4 mM. The energy-levels of the mismatched duplex shift below the energy level of the 100% complementary control at high concentrations of the chaotropic agent.

Figure 11 - shows the energy shift in the absence or presence of a "influencing" molecule, in this case spermidine. The molecular probe: (SEQ ID No. 4) 5'-GTG AGT TTG GGG AAA AAA AAT AAA ATA AAA ATG GCT TTC C-3' + Rhodamine 6G at 5'; The target sequence: (SEQ ID No. 5) 5'-GGA AAG CCA TTT TTA TTT TAT TTT TTT TCC CCA AAC TCA C-3'; Spermidin 5mM.

Experiments

**Example 1:**

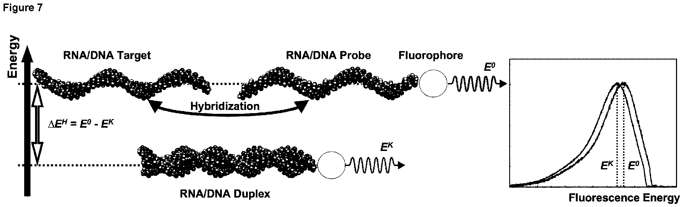

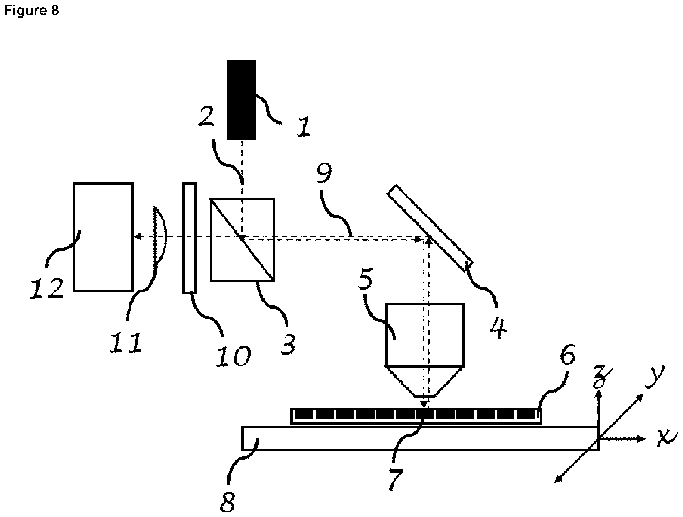

Schematically the experiment is shown on the Fig.7, where the molecular probe labelled with the fluorophore molecule under optical excitation will emit light spectrum with peak position at the energy E°. Adding into reaction target sequence will change emission spectrum and shift the emission spectrum to the new position $E^K$. To measure the spectra E° and $E^K$, the setup shown on the Fig.8, can be used. Measured spectra to be processed, subtracted background and fitted with a function comprising double Gauss distributions. First gauss corresponds to emission of the dye molecule in monomer state (higher energies) and second in dimer state (low energies). For further analysis only the line (gaussian line) for the dye in monomer state will be used. The line position $E^K$ will be analysed in function of time.

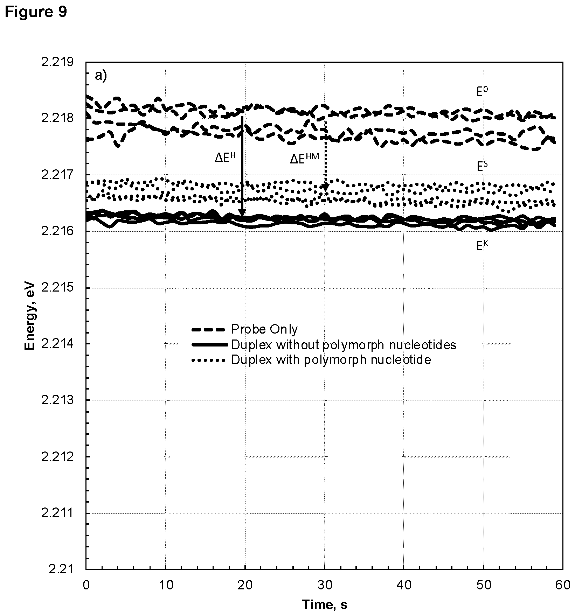

On the Fig.9a) are shown the result of hybridization of the two types of the samples. First, 0.3 μM) of 5'-AGA CCA GAA GAT CAG GAA CTC TA -3' molecular probe labeled with Rhodamine 6G dye hybridized with 0.3 μM perfectly matching target sequence 5'- TA GAG TTC CTG ATC TTC TGG TCT-3'. And second type of the samples, where the same molecular probe hybridized with 0.3 μM of the target sequence comprising single polymorph nucleotide 5'-TA GAG TTC CTG AT**T** TTC TGG TCT-3'. The hybridization reaction was carried in 50 mM Tris-HCl (pH 7.4) buffer. At first the only probe emission was measured dashed lines on the Fig.9a). After adding target sequences (perfectly matching and comprising polymorph nucleotide) the ingredients were incubated for at least 30 minutes at room temperatue. The emission (fluorescence) spectra of the incubated samples was measured for at least 60 sec with the rate of one spectrum per second. For each measured spectrum line position or emission energy was evaluated. Obtained dependencies are shown on the Fig.9a) with solid $E^K$ and doted $E^S$ lines. As. it can be seen incubation of the molecular probe with perfectly matching target and with the target comprising single polymorph nucleotide results in the fluorescence energy change. The energy difference between the only molecular probe and the probe reach of $\Delta E^H$~1.8meV or 21K for the perfectly matching target sequence and $\Delta E^{HM}$~1.6meV or 18K for the target with polymorph nucleotide.

Obtained result shows that adding target sequences, which must increase free-energy of the sample, in spite to that, leads to decrease of the energy. The energy decrease is stronger for the perfectly matching target and slightly less for the target with polymorph nucleotide. The observation can be explained by hybridization of the molecular probe with the two targets, where duplex formed with perfectly matching target results in formation of more stable state comparably with the case where the duplex is formed with the target comprising single polymorph nucleotide. Other words, to the duplexes the perfect duplex must be heated to higher temperatures. The observation well agrees with theoretical models describing molecular hybridization.

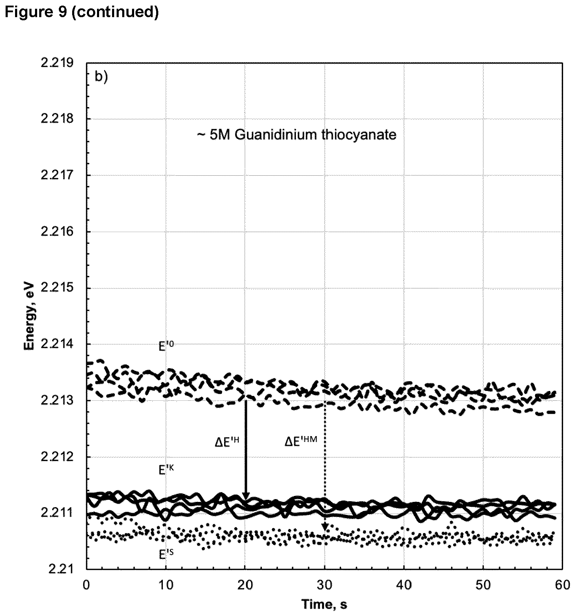

The same experiment was carried in the lysis buffer containing ~5 M guanidinium thiocyanate, 50 mM Tris-HCl, 22 mM EDTA and 1.2% Triton X-100, and the pH is 7.0 - 7.5, see Fig.9b). Similarly as before, fluorescence of the only molecular probe in the lysis buffer was measured at first $E^{0'}$. Afterwards the two types of the samples were created, first incubated with perfectly matching target sequence and second type where the molecular probe was incubated with the target comprising single polymorph nucleotide $E'^K$ and $E'^S$. Similarly, as before, fluorescence energy changed its position, however the change for the imperfect duplex $\Delta E'^{HM}$ ~ 2.3meV was stronger comparably with the perfect one $\Delta E'''$ ~ 2.0meV. The result is totally unexpected, because it shows that the less stable state become energetically favorable as the most stable duplex state. The possibility that the imperfect state may occupy lower energies as the perfect duplex state was already theoretically predicted. The free-energy difference between these states is small due to enthalpy-entropy compensation (Lumry R, Rajender S. Enthalpy-entropy compensation phenomena in water solutions of proteins and small molecules: a ubiquitous properly of water. Biopolymers: Original Research on Biomolecules. 1970 Oct;9(10):1125-227.) observed in aqueous solutions. Exclusion of the water and substitution it with the 5M guanidinium thiocyanate as well as the broken hydrogen bond in the non-matching pair leads to increase of the entropy of the duplex which is not compensated. This leads to favoring the imperfect state over the DNA duplex formed with perfectly matching target.

**Example 2:**

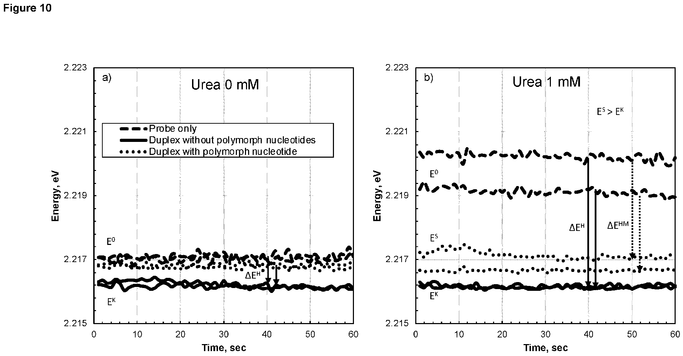

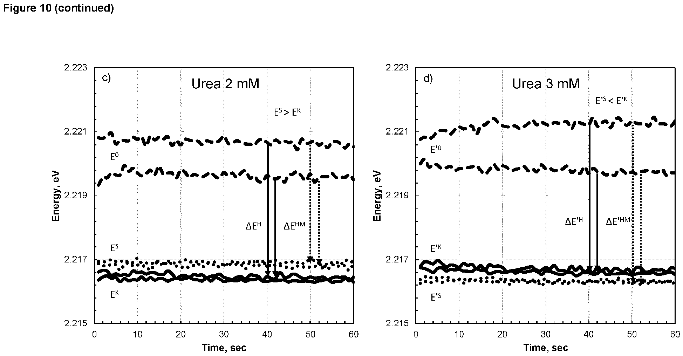

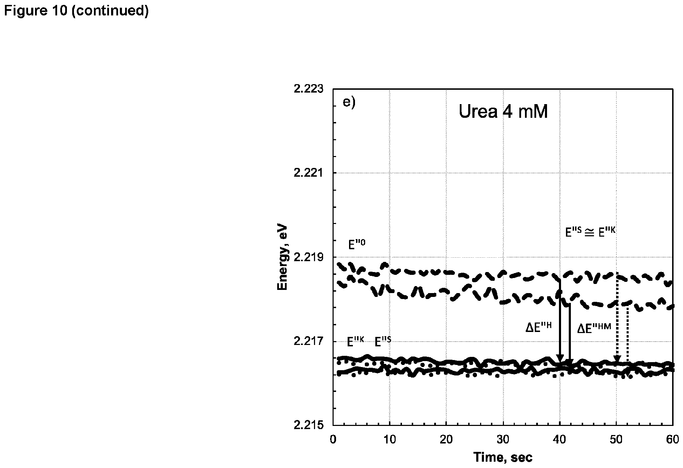

Another example is shown. on the Fig, 10. Here, hybridization of 0.3 μM of 5'-AGA CCA GAA GAT CAG GAA CTC TA -3' molecular probe and 0.3 μM perfectly matching target sequence 5'-TA GAG TTC CTG ATC TTC TGG TCT-3', as the first type of samples and the same molecular probe hybridized with 0.3 μM of the target sequence comprising single polymorph nucleotide 5'-TA GAG TTC CTG AT**T** TTC TGG TCT-3' as the second type of samples were performed in buffers comprising 0 mM, 1 mM, 2 mM, 3 mM and 4 mM of urea, Fig 10 a)-e) respectively. Previously as before experiment was started by measuring the molecular probe fluorescence energy in the respective buffers, afterwards the strands were added into the respective set of the samples. For the 0mM of urea, Fig 10a), formation of the imperfect duplex was not observed also perfect duplex showed less energy change ~1meV comparably with the Tris buffer $\Delta E^H$ ~1.8meV. Adding 1mM and 2mM of the urea, Fig. 10 b) and c) respectively shifted fluorescence energy of the samples comprising only the molecular probe into higher energies. Adding of the target sequenced (perfectly matching and containing single mismatching nucleotide) and >30 min incubation, resulted in significant lowering of the fluorescence energy $E^K$ and $E^S$ respectively. Here the perfect duplexes occupying lower energies comparably with the imperfect once, $E^S > E^K$. At around 3mM of urea concentration, the energy of the molecular probe only continues to grow but for the hybridized samples the situation changes, see Fig. 10d). The imperfect duplexes observed at lower energies as the perfect once $E'^S < E'^K$, apparently this can be considered as the point where the water was completely substituted with the urea molecules. Father increase of the urea concentration to 4mM, lead to decrease of the only probe energy comparable with the lower concentrations, as well as to the inability to resolve perfect and imperfect duplexes $E''^S \cong E''^K$.

**Example 3:**

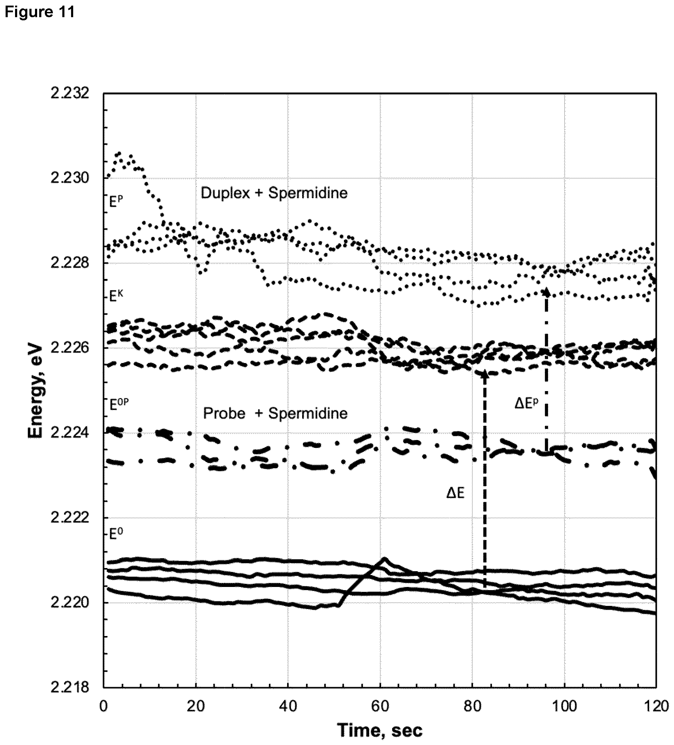

The last example is shown on the Fig. 11. Here, hybridization of the molecular probe (40 bp long, SEQ ID No. 6) 5'-GTG AGT TTG GGG AAA AAA AAT AAA ATA AAA ATG GCT TTC C-3' labeled with Rhodamine 6G with perfectly matching target 5'-GGA AAG CCA TTT TTA TTT TAT TTT TTT TCC CCA AAC TCA C-3' sequence SEQ ID No. 7 in 50 mM Tris-HCl (pH 7.4) buffer was studied. As it can be seen the only molecular probe fluorescence occurring in higher energies comparably to the fluorescence of the 23 pb's long probe, Fig. 9a), $E°$ ~ 2.221 eV (Fig. 11 solid line) vs. 2.218 eV respectively, this can be explained that adding longer nucleotides into the buffer causes significant perturbation for the buffer consequently the equilibrium between the buffer and the molecular probe will be reached at higher energies. Administering of the 0.3μM of complementary target into the samples causes further grow of the fluorescence energy to the $E^K$ -2.226 eV, dashed line. The observed energy values can be used to estimate of each individual state especially such which are not possible to measure, for example the ground state of the buffer $E^B$. For the case of the only molecular probe in the Tris the energy equilibrium equation can be written as:

$$E^B + E^0 = 2.221 \text{ eV}, \qquad (1)$$

Infusing of the same amount of the complementary target (the same length as the molecular probe), should double the $E°$, however it also triggers hybridization reaction, which decreasing the total energy on $\Delta E''$, so the energy equilibrium equation will look like:

$$E^B + 2E^0 - \Delta E^H = 2.226 \text{ eV}, \qquad (2)$$

where $\Delta E''$ can be estimated theoretically and for the duplex it may reach ~ 4.7meV. Solving system of (1) and (2) equation estimated buffer energy is $E^B$ -2.211 eV and adding to the buffer 0.3μM of the molecular probe increase the free energy on ~10 meV.

Finally, the similar measurements were performed in presence of 5mM spermidine. It is known that the spermidine and some other polyamines prefer to interact or bond to certain sites in DNA and also leading to conformational changes of the DNA. As it can be seen 5mM of the spermidine changing fluorescence energy of the molecular probe $E^{0P}$ -2.2235 eV, dash-dotted line, as well as the position of duplex also is changed and shifted into higher energies to $E^P$ ~2,228 eV. Repeating previous consideration, it is easy to conclude that the effective energy of the buffer comprising spermidine could be ~ 2.2143 eV and the duplex melting temperature will be on -5K higher as for the duplex formed without spermidine. The conclusion agrees with the observation made from the Tm measurements. Although the method investigating energy states nucleic acids interacting with the molecules (polyamines and/or proteins) is disclosed for the first time

**Claims**

1. A method for detecting nucleic acid hybridization, comprising the steps of:

a) measuring the energy ($E^0$) of a probe in a buffer, comprising the steps of

a1) providing a buffer medium;
a2) providing a nucleic acid probe with a fluorescence-label within that buffer medium;

a3) measuring the energy ($E^0$) of that system by exciting the fluorescence-tag and measuring the fluorescence of the system;

b) measuring the energy ($E^K$) of the probe hybridized to a control nucleic acid, comprising the steps of:

b1) using the same buffer medium of step a);
b2) providing the probe of step a2) and a control nucleic acid molecule which is 100% complementary to the nucleic acid probe;
b3) letting the probe and the complementary nucleic acid molecule hybridize at a temperature Ta which is from 0°C to the melting temperature of both molecules, preferably from above 0°C and up to 5°C below the melting temperature of both molecules;
b4) measuring the energy ($E^K$) of that system by exciting the fluorescence-tag and measuring the fluorescence of the system;

c) measuring the energy ($E^S$) of the probe hybridized to a sample nucleic acid, comprising the steps of:

c1) using the same buffer medium of steps a) and b);
c2) providing the probe of steps a2) and b2) and a sample nucleic acid molecule;
c3) letting the probe and the sample nucleic acid molecule hybridize at the same temperature Ta as used in step b3);
c4) measuring the energy ($E^S$) of that system by exciting the fluorescence-tag and measuring the fluorescence of the system;

and optionally,
d) repeating steps a) - c) in a buffer with a chaotropic agent; thereby receiving the energies $E'^0$, $E'^K$ and $E'^S$.
e) calculate the energy-shifts, comprising the steps of:

e1) calculate $\Delta E''$ according to the formula $\Delta E^H = E^0 - E^K$;
e2) calculate $\Delta E^{HM}$ according to the formula $\Delta E^{HM} = E^0 - E^S$;
e3) calculate $\Delta E'''$ according to the formula $\Delta E''' = E'^0 - E'^K$;
e4) calculate $\Delta E'^{HM}$ according to the formula $\Delta E'^{HM} = E'^0 - E'^S$;
e5) calculate $\Delta\Delta E$ according to the formula $\Delta\Delta E = |\Delta E^H| - |\Delta E^{HM}|$;
e6) optionally, calculate $\Delta\Delta E'$ according to the formula $\Delta\Delta E' = |\Delta E'^{HM}| - |\Delta E'^H|$;

wherein $|\Delta E^H| > |\Delta E^{HM}|$ - means that there is at least a single mispaired base-pair in the duplex of probe and sample nucleic acid;
wherein $\Delta\Delta E$ means a decrease of the duplex melting temperature;
wherein, optionally, $|\Delta E'^H| < |\Delta E'^{HM}|$ means that in the duplex are mispaired base-pairs and
wherein $\Delta\Delta E'$ can be used to calculate the number of mispaired base-pairs by using the formula $\Delta\Delta E' \sim n$ (Tm-Ta) $\Delta S$, where n is the number of mispaired base pairs, Tm - is the melting temperature, $T_a$ is the same temperature as of step b3), and $\Delta S$ is the entropy of a single broken hydrogen bond.

2. The method according to claim 1, wherein instead to the described steps c) to e), steps cc) and dd) are performed, comprising:

cc) repeating step b) in the presence of a sample molecule selected from a small molecule, peptide or protein, thereby measuring the energy $E^{0P}$ which is the energy of the free probe in buffer in the presence of the sample molecule and energy $E^P$ which is the energy of the hybridized nucleic acid molecule in the presence of the sample molecule;
dd) calculate $\Delta E$ according to the formula $\Delta E = E^K - E^0$,
ee) calculate $\Delta E^P$ according to the formula $\Delta E^P = E^P - E^{0P}$,
wherein the difference between $\Delta E$ and $\Delta E^P$ indicates an interaction of the sample molecule with the duplex of the probe and the sample nucleic acid molecule.

3. The method according to any of claims 1 or 2, wherein the buffer medium is water or any buffer allowing a hybridization of the nucleic acid molecules.

4. The method according to claim 3, wherein the buffer medium may comprise:

a) a salt selected from sodium chloride (NaCl), sodium citrate, and sodium phosphate, as well as any combination thereof;

b) a detergent selected from the group consisting of sodium dodecyl sulfate (SDS), polyethylene glycol tert-octylphenyl ether (Triton X-100), nonylphenoxypoly(ethyleneoxy)ethanol (Nonidet P-40 (NP-40)), octylphenoxypolyethoxyethanol (Igepal CA-630), Polysorbate 20 (Tween 20), 3-[(3-cholamidopropyl)dimethylammonio]-1-propanesulfonate (CHAPS), N-lauroylsarcosine sodium salt (Sarkosyl) and sodium deoxycholate (Deoxycholate), as well as any combination thereof;

c) a blocking agent selected from Denhardt's solution (comprising Ficoll, polyvinylpyrrolidone, and bovine serum albumin), salmon sperm DNA and herring sperm DNA, as well as any combination thereof;

d) a buffer such as Tris-HCl-buffer, HEPES-NaOH, saline-sodium citrate buffer, saline-sodium phosphate-EDTA buffer, as well as any combination thereof;

e) a chelating agent such as EDTA; and has a pH from 7.0 to 8.0.

5. The method according to any of the previous claims, wherein the buffer medium is selected from the group consisting of standard SSC (Saline-Sodium Citrate) buffer, Denhardt's solution, Church buffer, formamide hybridization buffer, DIG Easy Hyb, HEPES buffer, and a lysis-buffer, as well as combinations thereof.

6. The method according to any of the previous claims, wherein the nucleic acid of the probe, the control nucleic acid and/or the sample nucleic acid is selected independently from each other from the group consisting of DNA, RNA, PNA, XNA, LNA, morpholino oligomers, 2'O-Methyl-RNA, phosphorothioate DNA or RNA, guanosine-rich oligonucleotides, and D-amino acid-based nucleic acids, as well as combinations thereof.

7. The method according to any of the previous claims, wherein the fluorescence-label is is selected from the group consisting of fluorescent dyes such as DAPI, ethidium bromide, SYBR Green, Alexa Fluor dyes, and CyDyes, Quantum Dots and lanthanide-based labels, as well as combinations thereof.

8. The method according to any of the previous claims, wherein the chaotropic agent selected from guanidinium chloride (GuHCl), guanidinium thiocyanate (GuSCN), phenol, formamide, urea, potassium ions ($K^+$), lithium ions ($Li^+$), and magnesium ions ($Mg^{2+}$), as well as any combination thereof.

9. The method according to any of the previous claims, wherein the probe is excited by light in the wavelength corresponding to the absorption wavelength of the fluorescence-label.

10. The method according to claim 9, wherein the excitation light is provided by light-source selected from the group consisting of a mercury arc lamp, a xenon arc lamp, an LED, a LASER, a metal halide lamp, a tungsten-halogen lamp, and a deuterium lamp, as well as combinations thereof.

11. The method according to any of the previous claims, wherein the mispairing is a pairing selected from the group consisting of A-A, A-C, C-A, A-G, G-A, T-T, T-C, C-T, T-G, G-T, C-C, G-G, U-U, A-U, U-A, as well as combinations thereof,
wherein A is adenosine, C is cytosine, T is tyrosine, G is guanine, and U is uracil.

12. The method according to any of the previous claims, wherein the sample nucleic acid molecule may be derived from a cell-lysate, a biological sample such as a tissue sample, or a body fluid selected from the group consisting of blood, urine, saliva, sweat, lymph fluid, cerebrospinal fluid (CSF), gastric juice, pleural fluid, peritoneal fluid, synovial fluid (joint fluid), and sputum (phlegm), as well as combinations thereof.

13. The method according to claim 2, wherein the interference is selected from the group consisting of bending of the nucleic acid, looping of the nucleic acid, altering accessibility and compaction of the nucleic acid, unwinding of the nucleic acid, supercoiling the nucleic acid, and modify the nucleic acid, as well as combinations thereof.

14. The method according to any of the previous claims, for use in diagnostics outside the human or animal body and/or in medicine.

15. The method according to any of the previous claims, wherein the sample nucleic acid molecule is a nucleic target for use in the detection of a nucleic acid mispairing in diagnostics, research, or a therapeutic application in a biological and/or medical field.

**Figure 1**

Schematic depiction of steps a) and b)

$E^0$ — a1-3)

$E^K$ — b1-4)

$\Delta E^H$

Energy (eV)

time

a1-3) — Fluorescent Dye — Nucleic acid probe

b1-4) — Fluorescent Dye — Nucleic acid probe — Nucleic acid 100% complementary

**Figure 2**

**Figure 3**

Schematic depiction of step d)

$\Delta E'^{H}$

$\Delta E'^{HM}$

$E'^{K}$

$E'^{S}$

*chaotropic buffer

Energy (eV)

time

d1-4)

Fluorescent Dye

Nucleic acid probe

Sample nucleic acid w. misparings

Chaotropic agent

Figure 4

Schematic depiction of ΔΔE

Figure 5

Schematic depiction of different energy-levels

Figure 6

Schematic depiction of steps cc) – ee)

+ „influencing molecule"

EP 4 686 762 A1

Figure 7

30

**Figure 8**

**Figure 9**

Figure 9 (continued)

Figure 10

Figure 10 (continued)

Figure 10 (continued)

Figure 11

Europäisches
Patentamt
European
Patent Office
Office européen
des brevets

# EUROPEAN SEARCH REPORT

**Application Number**

EP 24 19 1540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/209565 A2 (ICHORTEC GMBH [DE]) 21 October 2021 (2021-10-21) | 1 | INV. C12Q1/6827 C12Q1/6816 |
| Y | * claim 1; p. 11, 3rd para.; p. 14, 2nd para. - p. 15, 2nd para.; para. bridging p. 18-19; fig. 4-6 * | 1-15 | |
| Y | NIMSE SATISH BALASAHEB ET AL: "A Novel Method That Allows SNP Discrimination with 160:1 Ratio for Biosensors Based on DNA-DNA Hybridization", BIOSENSORS, vol. 11, no. 8, 6 August 2021 (2021-08-06) , page 265, XP093237295, CH ISSN: 2079-6374, DOI: 10.3390/bios11080265 * p. 1, last para. * | 1,3-12, 14,15 | |
| Y | K. GRACIE ET AL: "Interaction of fluorescent dyes with DNA and spermine using fluorescence spectroscopy", ANALYST, vol. 139, no. 15, 22 May 2014 (2014-05-22) , pages 3735-3743, XP055544618, UK ISSN: 0003-2654, DOI: 10.1039/C4AN00680A * abstract * | 2-15 | |
| A | SAITO YOSHIO ET AL: "An environmentally sensitive fluorescent purine nucleoside that changes emission wavelength upon hybridization", CHEMICAL COMMUNICATIONS, vol. 49, no. 50, 1 January 2013 (2013-01-01), page 5684, XP093237370, UK ISSN: 1359-7345, DOI: 10.1039/c3cc42605j * the whole document * | 1-15 | **TECHNICAL FIELDS SEARCHED (IPC)** C12Q |

-/--

The present search report has been drawn up for all claims

| Place of search | Date of completion of the search | Examiner |
|---|---|---|
| Munich | 9 January 2025 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

EP 4 686 762 A1

European Patent Office — Europäisches Patentamt — European Patent Office — Office européen des brevets

# EUROPEAN SEARCH REPORT

Application Number

EP 24 19 1540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HWANG GIL TAE: "Single-Labeled Oligonucleotides Showing Fluorescence Changes upon Hybridization with Target Nucleic Acids", MOLECULES, vol. 23, no. 1, 8 January 2018 (2018-01-08), page 124, XP093237120, CH ISSN: 1420-3049, DOI: 10.3390/molecules23010124 * the whole document * | 1-15 | |
| A,D | LUMRY RUFUS ET AL: "Enthalpy-entropy compensation phenomena in water solutions of proteins and small molecules: A ubiquitous properly of water", BIOPOLYMERS, vol. 9, no. 10, 1 October 1970 (1970-10-01), pages 1125-1227, XP093236696, Hoboken, USA ISSN: 0006-3525, DOI: 10.1002/bip.1970.360091002 Retrieved from the Internet: URL:https://onlinelibrary.wiley.com/doi/10.1002/bip.1970.360091002> * the whole document * | 1-15 | TECHNICAL FIELDS SEARCHED (IPC) |

-/--

The present search report has been drawn up for all claims

| Place of search | Date of completion of the search | Examiner |
|---|---|---|
| Munich | 9 January 2025 | Ripaud, Leslie |

EPO FORM 1503 03.82 (P04C01)

page 2 of 3

Europäisches
Patentamt

European
Patent Office

Office européen
des brevets

**EUROPEAN SEARCH REPORT**

Application Number

EP 24 19 1540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MOONEY JONATHAN ET AL: "Get the Basics Right: Jacobian Conversion of Wavelength and Energy Scales for Quantitative Analysis of Emission Spectra", JOURNAL OF PHYSICAL CHEMISTRY LETTERS, vol. 4, no. 19, 3 October 2013 (2013-10-03), pages 3316-3318, XP093237556, US ISSN: 1948-7185, DOI: 10.1021/jz401508t * the whole document * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 9 January 2025 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 1540

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2025

| Patent document cited in search report | Publication date | Patent family member(s) | Publication date |
|---|---|---|---|
| WO 2021209565 A2 | 21-10-2021 | EP 3896430 A1<br>US 2021324455 A1<br>WO 2021209565 A2 | 20-10-2021<br>21-10-2021<br>21-10-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 20169908
- EP 2022083446 W
- DE 102019132525

**Non-patent literature cited in the description**

- **SANTALUCIA J**. A unified view of polymer, dumbbell, and oligonucleotide DNA nearest-neighbor-thermodynamics. *Proceedings of the National Academy of Sciences*, 1998, vol. 95 (4), 1460-1465
- **PETRUSKA J** ; **SOWERS LC** ; **GOODMAN MF**. Comparison of nucleotide interactions in water, proteins, and vacuum: model for DNA polymerase fidelity. *Proceedings of the National Academy of Sciences*, March 1986, vol. 83 (6), 1559-62
- **SANTALUCIA J JR**. *Proc. Natl. Acad. Sci. USA*, 1998, vol. 95
- **TYAGI S** ; **KRAMER FR**. Molecular beacons: probes that fluoresce upon hybridization. *Nat. Biotechnol.*, 1996, vol. 14 (3), 303-308
- **LUMRY R** ; **RAJENDER S**. Enthalpy-entropy compensation phenomena in water solutions of proteins and small molecules: a ubiquitous properly of water. *Biopolymers: Original Research on Biomolecules*, October 1970, vol. 9 (10), 1125-227